(19) Europäisches Patentamt
European Patent Office
Office européen des brevets

(11) EP 2 032 201 B1

(12) EUROPEAN PATENT SPECIFICATION

(45) Date of publication and mention of the grant of the patent:
**03.04.2013 Bulletin 2013/14**

(21) Application number: **07748192.7**

(22) Date of filing: **31.05.2007**

(51) Int Cl.:
***A61N 1/05*** ^(2006.01)^ ***A61M 25/00*** ^(2006.01)^
***B29C 53/52*** ^(2006.01)^ ***H05K 1/00*** ^(2006.01)^

(86) International application number:
**PCT/SE2007/000528**

(87) International publication number:
**WO 2007/139479 (06.12.2007 Gazette 2007/49)**

(54) **TUBULAR CATHETER FOR INVASIVE USE AND MANUFACTURING METHOD THEREFOR**

ROHRFÖRMIGER KATHETER FÜR INVASIVE VERWENDUNG UND HERSTELLUNGSVERFAHREN DAFÜR

CATHETER TUBULAIRE POUR UTILISATION INVASIVE ET PROCEDE DE FABRICATION DE CELUI-CI

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **01.06.2006 US 803658 P**

(43) Date of publication of application:
**11.03.2009 Bulletin 2009/11**

(73) Proprietor: **Cathprint AB**
**187 35 Täby (SE)**

(72) Inventors:
- **KÄLLBÄCK, Bengt**
**S-187 35 Täby (SE)**
- **HULT, Anders**
**S-187 34 Täby (SE)**
- **BRODIN, Lars-Åke**
**S-187 35 Täby (SE)**
- **ELMQVIST, Håkan**
**S-167 62 Bromma (SE)**

(74) Representative: **Holmberg, Martin Tor**
**Bergenstrahle & Lindvall AB**
**P.O. Box 17704**
**118 93 Stockholm (SE)**

(56) References cited:
**EP-A2- 1 188 976**
**WO-A-2005/025401**
**US-A- 2 679 195**
**US-A- 4 762 135**
**US-A- 6 024 702**
**US-A1- 2004 238 529**
**US-A1- 2005 038 320**
**US-A1- 2005 038 320**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 032 201 B1

## Description

The present invention relates to the field of devices for invasive use.

BACKGROUND

A number of different devices for invasive use are known, but in several cases their performance is not entirely satisfactory.

EP-A1-1 714 610 shows a catheter wherein a flexible printed circuit board is mounted in a tube. An electronic component is mounted on the flexible printed circuit board. This catheter is relatively complex which makes manufacturing relatively expensive and which also can have a negative impact on reliability.

In US-A-4 762 135 there is shown a cochlea implant in the form of a single-sided flexible printed wire board rolled into a tube shape. This implant is not suitable as an instrument that will be in contact with body fluids since there is a risk that the electrical conductors on the outside of the implant would be short circuited by such body fluids.

In the article "Die separation and packaging of a surface micromachined piezoresistive pressure sensor" by Ingelin Clausen and Ola Sveen, published in Sensors and Actuators A: Physical, Volume 133, Issue 2, 12 February 2007, Pages 457-466, the use of a pressure sensor for invasive use is discussed. The pressure sensor is mounted on a flexible printed circuit board which is placed in a silicone rubber catheter with the sensor inside the tip of the catheter and the flexible printed circuit board being integrated into the wall of the catheter. This catheter is rather complex which makes the manufacturing quite expensive and which also can have a negative impact on its reliability.

US-A1-5 199 433 shows an esophageal catheter comprising a structure with a flexible printed wire board having electrodes and a sensor.. The structure is attached to a probe using adhesive. This construction is not suitable for invasive applications since it has a relatively large diameter. It can as well be cumbersome to use, since the structure has to be attached to the probe before use.

In US-A-5 902 330 there is shown a lead for a cardiac pacemaker wherein the stimulation electrode is glued to a supporting body.

In the article "Continuous stroke volume and cardiac output from intra-ventricular dimensions obtained with impedance catheter", Cardivascular Research, 1981, 15, 328-334, Baan J. et al., a method of measuring volume and pressure in the left ventricle of the heart is disclosed. Electrodes on the catheter are used for the impedance measurement, typically 10-12 electrodes. However, the catheters known from the background art are too stiff and thick to be suitable for clinical use in this method. They may for example cause arrhythmias.

In the doctoral thesis "New approaches to monitoring of cardiac function", Emil Söderqvist, 2006, Division of Medical Engineering, Karolinska Institute, KTH School of Technology and Health, ISBN-10: 91-7178-507-8, there is presented a method of measuring volume and pressure in the left ventricle of the heart similar to the one presented in the Baan et al. article cited above, but where only four electrodes are needed.

To the best of our knowledge there is also no rational production process available for the kind of devices (catheters) that are discussed above. In general the catheters are long (in the order of one meter) and thin (between 0,3 and 3mm) and are composed of several electrical conductors and a guiding structure. These conductors are usually insulated wires often as thin as about 20-30 micrometer. The guiding structure is often a flexible tube in which case the thin fragile wires need to be inserted and pulled through the entire tube or some other structure where the wires are attached. In both cases cumbersome and critical (from the perspective of production quality and efficiency) handling is needed to assemble the catheters. The production is even more delicate when measuring devices should be incorporated in the catheter. In such cases mounting of the measuring device often needs to be done in a separate tube and the thin wires pulled all the way through the longer tube. The thin wires are often wire bonded to the measuring device (chip). Finally the tubes are joined and the wires are pulled back and attached to electrodes in the rear end of the catheter. The attachments of the wires to the electrodes are often difficult since the wires are very thin and the space is limited where the wires are attached to the electrodes. Such a catheter often consists of four different tube sections that are joined together.

Present production methods may lead to devices with unsatisfactory performance. For example catheters for simultaneous measuring of pressure and volume in the ventricles of the heart are in some cases so thick and stiff that they may cause arrhythmia and leakage through the valves.
Document US-A-2005/038320 discloses the most relevant prior art.

SUMMARY OF THE INVENTION

It is an object of the present invention to provide a device for invasive use that is improved in comparison to the known devices.

The invention is defined in claim 1.

In one embodiment the support member is at least partly filled with a flexible resilient material, such as an adhesive or a polymer.

In another embodiment the support member is completely filled with a flexible resilient material, such as an adhesive or a polymer.

In a further embodiment the inside of the support member is completely sealed from the outside of the support member.

In yet another embodiment the device for invasive use is adapted to be provisionally located in a body by surgical invasion while or for monitoring or influencing the function of an organ.
In another embodiment the device for invasive use is adapted to be provisionally located in a body by surgical invasion while or for monitoring or influencing the function of a heart.
In another embodiment the adjacent edges of the support member are at least partly joined by welding.
In a further embodiment the adjacent edges of the support member are at least partly joined by an adhesive.
In yet another embodiment the at least one sensing, stimulating and/or processing element comprises at least one electronic component or microelectromechanical system, provided on the inside of the at least partly tube shaped support member.
In one embodiment the at least one electronic component or microelectromechanical system is chosen among a pressure sensor, a voltage sensor, a pH sensor, a temperature sensor, a gas sensor, a component for detecting or quantifying a reagent (for example a protein), and a drug delivery device.
In another embodiment at least one electrode is placed on the outside of the at least partly tube shaped support member.
In yet another embodiment at least one electrode is placed on the inside of the at least partly tube shaped support member.
In a further embodiment at least four electrodes are provided on the device for invasive use, said at least four electrodes constituting a volume sensor.
In one embodiment there is provided at least one reinforcing or rigidifying element on the inside of the support member.
In another embodiment the at least one reinforcing or rigidifying element extends beyond one or both ends of the support member.
In yet another embodiment the at least one reinforcing or rigidifying element comprises an optical fibre or waveguide.
In a further embodiment there is provided at least one optical fibre or waveguide on the inside of the support member.
In another embodiment the at least one electronic component or microelectromechanical system is placed adjacent a front end of the support member and in operational contact with the at least one electrically conductive line or pattern or said at least one optical fibre or waveguide.
The following manufacturing methods are not part of the invention.
Generally, a method for manufacturing a device for invasive use, may comprise the steps of;

- providing a support member comprising a flexible material.
- providing a tool or jig comprising at least one hole therein that is at least partly conical or funnel-shaped. The tool or jig further comprises entrance means for keeping an adhesive material in, or bringing an adhesive material to, a liquid state. The tool or jig further comprises exit means for solidifying the adhesive material.

The method may further comprise the steps of:

- feeding the support member through the hole or through hole so as to at least partly form the support member into a tube shape.
- applying an adhesive material to the support member as it is being fed through the hole in the tool or jig, whereby the adhesive material is continuously solidified as the support member is being fed through the hole in the tool.
- selecting an adhesive material with sufficient adhesive strength to keep the support member in a tube shape.

In one embodiment the method for manufacturing further comprises the step of filling the support member with adhesive material as the support member is being fed through the hole in the tool or jig.
In another embodiment the method for manufacturing further comprises the step of at least partly joining the adjacent edges of the support member to each other by means of the adhesive material. The adhesive material is applied to at least one of.the adjacent edges of the support member.
In yet another embodiment the method for manufacturing further comprises the step of heating the entry area of the hole or through hole. The entry area is heated with the entrance means so as to heat the adhesive material. Further, the exit area of the hole is cooled with the exit means so as to solidify the adhesive material.
Alternatively, a method for manufacturing a device for invasive use may comprise the steps of:

- providing a support member comprising a flexible material.
- providing a tool or jig comprising at least one hole therein that is at least partly conical or funnel-shaped.
- feeding the support member through the hole so as to at least partly form the support member into a tube shape.
- welding the adjacent edges of the support member to each other.

There is also provided a method of using the device for invasive use. The device for invasive use may be in any of the embodiments described above. The device for invasive use may for example be used diagnostically or therapeutically.
In another embodiment of the method of using the device for invasive use, said device is used for monitoring or influencing the function of a heart.

BRIEF DESCRIPTION OF THE DRAWINGS

Fig. 1 is a schematic drawing showing one example of the device 100 for invasive use,
Fig. 2 is a detailed view of an electrode showing a via hole 121 and a via conductor 123,
Figs. 3a, 3b are drawings showing one embodiment of the device 100 for invasive use,
Fig. 4 is a cross section of one embodiment of the device 100 for invasive use,
Figs. 5a, 5b are figures showing the back end of one embodiment of the device 100 for invasive use,
Figs. 6a, 6b are detailed views showing different methods of mounting a pressure sensor 201,
Fig. 7 is a drawing showing the front end of the device 100 for invasive use in one embodiment,
Fig. 8 is a drawing showing the manufacturing principle of the device 100 for invasive use,
Fig. 9 is a drawing showing one embodiment of a manufacturing tool,
Fig. 10 is a detailed view of the manufacturing tool,
Fig. 11 is a view showing the support member,
Figs. 12, 13 show different aspects regarding manufacturing,
Figs. 14-16 are different detailed views of the device 100 for invasive use,
Figs. 17a, 17b are drawings showing a prototype device of the device 100 for invasive use,
Fig. 18 is a detailed view showing the mounting of a pressure sensor 303 on the prototype device 300,
Fig. 19 is an overview of a system 700 including the device 100 for invasive use,
Figs. 20-22 show one example of the pressure sensor 201.
Figs. 23, 24 show examples of signals in the system 700.
Fig. 25 is a drawing showing the switches Switch_0 and Switch_90.

DETAILED DESCRIPTION OF THE INVENTION

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" also include plural referents unless the context clearly dictates otherwise. Thus, for example, reference to "a member" includes more than one such member, and the like.

In this specification and in the claims which follow, reference will be made to a number of terms which shall be defined to have the following meanings:

The term "about" is used to indicate a deviation of +/- 2 % of the given value, preferably +/- 5 % and most preferably +/- 10 % of the numeric values, when applicable.

With reference to the figures, the device 100 for invasive use, the manufacturing thereof and its use will now be described in an exemplary way.

With reference to Figs. 1-7, the device 100 for invasive use described herein is generally a long (often about 0,05-2,50m, advantageously about 0,1m - abut 0,3m or about 0,3m - about 1,8m), thin (diameter about 0,1-3mm, advantageously about 0,3mm - about 2mm or about 0,5mm - about 1,5mm) and hollow probe that is inserted into organs in the human body in order to support physicians through measurement, stimulation and/or affecting the body in other ways. The device 100 for invasive use may be inserted into the body in various ways, e.g. through blood vessels or directly through body tissue.

Different parameters may be measured, such as for example pressure (blood or tissue pressure), partial pressure of gases e.g. oxygen, temperature, flow velocity, pH and the presence, concentration, composition or other parameters regarding chemical substances. Chemical sensors, for example FET-based chemical sensors, may be used (Field Effect Transistor, FET). The diameter of an artery or vein, or the volume of a cardiac chamber may be measured, the presence of a stenosis may be discovered, oxygen saturation may be measured. Regarding stimulation or affecting, different organs such as for example the heart may be stimulated or affected. Different ways of stimulating or affecting may be used such as electrical signals, heat, infusion of chemical substances and mapping with ultra sound. Depending on the application the device 100 for invasive use may be longer or shorter. When the device 100 for invasive use is inserted through for example an artery or a vein it may be advantageous that the device 100 for invasive use is approximately 50-250 cm long. When the device 100 for invasive use is inserted through the aorta at the groin to reach the heart it may be advantageous that the device 100 for invasive use is about 120-240 cm long. When the device 100 for invasive use is introduced at the throat/cervix to reach the heart it may be advantageous that the device 100 for invasive use is about 50-100 cm long.

When the device 100 for invasive use is inserted into an organ directly through tissue it may be advantageous that the device 100 for invasive use is about 5-50 cm long, in some applications about 10-20 cm long. When the device 100 for invasive use is introduced into the heart directly through the cardiac wall or through myocardium it may be advantageous that the device 100 for invasive use is about 30-60 cm long._The front end 107 (inside the body) may comprise at least one front end electrode 111 that is in contact with the substance surrounding the device 100 for invasive use. The at least one front end electrode 111 may for example be used to measure voltages generated by nerve signals or by excitation electrodes. The front end 107 may as well comprise at least one passive and/or active electronic component or microelectromechanical system 200 on the inside of the device 100 for invasive use. Such an electronic component or microelectromechanical system 200 may e.g. comprise a pressure sensor 201, for example of the piezo electrical type, having contact with the surrounding body fluid through an access hole 115 in the device 100 for invasive use. The pressure sensor 201 may comprise a piezo electrical crystal. Another example may be a signal processing component, e.g. an amplifier used to amplify weak signals measured by the front end electrodes 111 or some other measuring element. For such a component there is no need for an access hole 115 to the surrounding body fluid. The at least one front end electrode 111 may also be used for stimulation with electrical voltage or current as in pacing applications or in the method described below, simultaneous measurement of pressure and volume of the heart's ventricles. Multiple electronic components and/or microelectromechanical systems 200 may be incorporated, it may for instance be advantageous to use several pressure sensors for diagnosis of stenosis in the coronary arteries to be able to measure the pressure on both sides of the stricture.

<u>General description of the construction and manufacturing of the device 100 for invasive use-described herein.</u>

The basis for the device 100 for invasive use is a flexible strip or foil, hereafter called support member 101. The support member 101 is advantageously long, thin and narrow and advantageously comprises a suitable insulating material, e.g. polyimide or a cycloaliphatic polyolefine.

One non-limiting example of the latter is the product Topas® COC (TOPAS Advanced Polymers GmbH, Oberhausen, Germany or Ticona GmbH, Kelsterbach, Germany). Advantageously the material for the support member 101 is biologically-inert or bio-compatible. The device 100 for invasive use may also be covered with a layer of biocompatible hydrogel on the outside to reduce the friction and for improved biocompatibility, e.g. to avoid that blood coagulates on the outside of the device 100 for invasive use. The measures of the support member 101 may in one advantageous embodiment be 100 cm long, 1.5 mm wide and 50 micrometer thick. But as described before, the length, as well as the thickness and the width, may vary depending on the application. The width may be in the interval of 0,5-5 mm, more advantageously 1-3 mm, and the thickness may be in the interval of 10-200 micrometer, more advantageously 30-70 micrometer, in one particular embodiment 50 micrometer is used. Generally a greater thickness results in a more rigid device 100 for invasive use and a smaller thickness results in a less rigid one. Now an embodiment will be described where electrically conductive lines and patterns 111, 113, 117 are arranged on both sides of the support member 101. At certain points there are holes, called via holes, 121 in the support member 101. The electrically conductive lines and patterns 111, 113, 117 on both sides of the support member 101 are electrically connected through the via holes 121. In the via holes 121 there are electrical conductors, via conductors 123, connecting the electrically conductive lines and patterns 111, 113, 117 on both sides of the support member 101. Advantageously the via conductors 123 comprise electrically conductive material on the walls of the via holes 121. The electrically conductive lines and patterns 111, 113, 117 may comprise a suitable metal, e.g. Copper or an electrically conductive polymer or another electrically conductive material. The support member 101 with the electrically conductive lines and patterns 111, 113, 117 placed thereon or attached thereto may be called a flexible printed wire board (flexible PWB) and the electrically conductive lines and patterns 111, 113, 117 can for instance be attached to the support member 101 with standard flexible printed wire board manufacturing equipment. When the support member 101 comprises layers of electrically conductive lines and patterns 111, 113, 117 on both sides thereof (as in the embodiment described here) it is hereafter called double sided support member. On a first side 125 of the support member 101 (the side that will become the "inside" of the device 100 for invasive use) there are electrically conductive lines or patterns 117 and on a second side 127 (the side that will become the "outside" of the device 100 for invasive use) there are electrically conductive areas or patterns 111, 113 that will serve as electrodes inside the body (front end electrodes 111) and as contacts to external electronic and data processing equipment (back end electrodes 113). The lines or patterns 117 on the first side 125 of the support member 101 connect the front end electrodes 111 and/or the at least one electronic component and/or at least one microelectromechanical system 200 with the back end electrodes 113. On the first side 125 of the support member 101 ("inside") at least one electronic component and/or at least one microelectromechanical system 200 may be mounted, it/they may for example be flip-chip mounted or wire-bonded. Conducting adhesive, soldering or any other suitable attachment method may be used. In some cases packaged chips can be used, but normally the chips are unpackaged ("naked") to save space. When the at least one electronic component and/or at least one microelectromechanical system 200 have been mounted, the support member 101 is at least partly rolled up into a tube and simultaneously filled with adhesive or glue 601 that holds the support member 101 in a tube shape. Formation of the support member 101 at least partly into a tube is advantageously done by feeding the support member 101 through a hole 609 with a funnel-like opening 611 where the circumference of the hole 609 matches the width of the support member 101. The width of the support member 101 substantially equals the circumference of the hole 609. The circumference of the hole 609 equals the diameter of the hole 609 times $\pi$. When a single sided support member 101 is used it is advantageous that the width of the support member 101 is the same as the circumference of the hole 609. When a double sided support member 101 is used it is advantageous that the width of the support member 101 is slightly smaller than the circumference of the hole 609. This is necessary because elements on the second side 127 of the support member 101, such as the electrically conductive areas or patterns 111, 113, needs some space in the hole 609. After feeding the support member 101 through the hole 609, the first 125 and second 127 side of the support member 101 have respectively become inside and outside of the at least partly tube shaped support member 101. An electronic component or microelectromechanical system 200 that is used for measurement or stimulation may be mounted over a hole or opening 115 in the support member 101 so as to have contact to the surrounding body tissue and/or fluids whereas signal processing components 200 are totally concealed. Parameters that may be measured include pressure, temperature, flow, pH, partial pressure of oxygen, mapping with ultra sound etc. It is also possible to combine different electronic components and/or microelectromechanical systems 200 to achieve multi functionality or to integrate several electronic components or microelectromechanical systems 200 of one kind to get extended functionality. One such example could be several pressure sensors 201 in order to improve diagnosis of stenosis in the coronary arteries.

Fig. 5 shows how a optical conductor 129 may be placed in the at least partly tube shaped device 100 for invasive use. The optical conductor protrudes from the back end of the device 100 for invasive use.

Fig. 6a shows in detail the mounting of a pressure sensor 201 according to one suitable method. The bond pads 201a-201c on the pressure sensor 201 are respectively attached to the bond pads 118a-118c on the support member. The bond pads may be attached to each other by for example soldering or conducting adhesive. The pressure sensitive area 201d of the pressure sensor 201 may comprise a pressure sensor membrane 201e. In addition or alternatively there may also be provided a support member membrane 131 mounted in the access hole 115 in the support member 101. The pressure sensitive area 201d of the pressure sensor 201 is placed in or over the access hole 115 in the support member 101.

Fig. 6b shows in detail the mounting of a pressure sensor 201 according to a method that may be advantageous since it enables a strong or rigid fastening or mounting of the pressure sensor 201. In this embodiment the bond pads 201a-201c are placed on the upper side of the pressure sensor 201. Said bond pads 201a-201c are electrically connected to the electrically conductive lines 207 on the underside of the pressure sensor 201 by means of via holes 203a-203c which are provided with via conductors 205a-205c. Advantageously the via conductors 205a-c comprise electrically conductive material on the walls of the via holes 203a-c. That means that almost the entire area (except the pressure sensitive area 201d and the via holes 203a-c) of the underside of the pressure sensor 201 can be used for mounting the pressure sensor 201 to the support member 101, e.g. by using an adhesive. This enables a strong fastening of the pressure sensor 201. The bond pads 118a-118c on the support member 101 are placed adjacent or next to the mounted pressure sensor 201. The bond pads 118a-118c on the support member 101 are connected to respective bond pad 201a-201c on the pressure sensor 201 e.g. by means of wire bonding (wires 209a-209c), e.g. by using gold wires. When the pressure sensor 201 is mounted and the bonding wires connected the pressure sensor 201 including the bonding wires 209 may be covered by e.g. silicone to protect the bonding wires and further strengthen the mounting of the pressure sensor 201.

One advantage of using a support member 101 comprising electrically conductive lines or patterns 111, 113, 117 on both sides is that the back end electrodes 113 can be placed on the outside of the at least partly tube shaped device 100 for invasive use. This is an advantage when using the back end electrodes 113 to connect the device 100 for invasive use to external electronic and data processing equipment. Placing the back end electrodes 113 on the outside enables an uncomplicated construction of the contact for connecting the device 100 for invasive use to external electronic and data processing equipment.

It is also possible to manufacture a device 100 for invasive use with the same functionality as described above but based on a multi layer concept where several support members 101, with one or two layers of electrically conductive lines and patterns 111, 113, 117 arranged thereon, are placed on top of each other.

It is as well possible to manufacture a device 100 for invasive use with the same functionality as described above but based on a single sided support member. That is, a support member with electrically conductive lines or patterns 111, 113, 117 on only one side. In this case the conductive area for the front end and back end electrodes is put on the inside of the at least partly tube shaped support member 101 and access to the electrodes from the outside is arranged by removing part of the support member 101, preferably by laser ablation (fig. 14). This approach has the advantage of avoiding fitting of electrically conductive patterns or lines 111, 113, 117 on both sides of the support member 101 which may be cumbersome over long distances.

The back end electrodes 113 may in this case also be contacted with means of a contact that is inserted inside the at least partly tube formed support member 101. Another possibility is to let the part of the support member 101 where the back end electrodes 113 are placed to remain flat; i.e. not to form this part into a tube (fig. 15). In this way the back end electrodes 113 may be contacted by means of a flat contact.

Another feature that may be advantageous is to provide the device 100 for invasive use with an effective shielding by adding a mesh of thin metal lines on the outside of the support member 101 (fig. 16). This may often be advantageous since signal levels (of the signals in the electrical conductors in the device 100 for invasive use) generally are low and the hospital environment often quite noisy electromagnetically. Noisy meaning that the level of electromagnetic radiation in general is relatively high in a hospital environment.

When the device 100 for invasive use is introduced into a canal, such as an artery or a vein, in the body it may be advantageous to introduce a catheter guide prior to the device 100 for invasive use which is then introduced inside the catheter guide. When the device 100 for invasive use is in its end position the catheter guide is withdrawn over the device 100 for invasive use. When the catheter guide is withdrawn it is necessary to hold the device 100 for invasive use so that it is not withdrawn together with the catheter guide. Consequently, the device 100 for invasive use has to be long enough (basically twice as long as needed from a clinical point of view) so that it can be held in position during the withdrawal of the catheter guide. However, the main part of the device 100 for invasive use that is outside of the body is only used for this purpose, to enable the device 100 for invasive use to be kept in position while the catheter guide is being withdrawn. Traditionally, devices for invasive use, such as catheters, have been manufactured as fully functional devices to their entire length. This may be disadvantageous since the part of the catheter being outside of the body often is relatively long, around 100 cm is not unusual, and may get damaged or be hindering when the patient is treated, examined or otherwise handled.

Due to the construction of the device 100 for invasive use described herein it is possible to manufacture the part of the device 100 for invasive use that is not needed from a clinical point of view as a "non-functional" part 135 (from a clinical point of view), a "dummy", in a convenient way. According to such an embodiment the support member 101 has the full length needed, but the back end electrodes 113 are placed close to the point where the device 100 for invasive use enters the body (or the point where the device 100 for invasive use protrudes from the body through the insertion hole). In this way the part of the device 100 for invasive use that extends behind the back end electrodes 113 may comprise merely the support member 101 and it may be cut off or otherwise separated from the rest of the device 100 for invasive use after the catheter guide has been withdrawn. The separation of the "non-functional" part 135 may be facilitated by means of for example a perforation 137. In this way the device 100 for invasive use only extends a short distance outside the body and the risk that the device 100 for invasive use should be hindering or damaged is reduced substantially.

To use a catheter guide is a commonly used procedure.

<u>Prototype device</u>

The device 100 for invasive use described herein has been verified by the design and manufacture of a prototype device 300 for the simultaneous measurement of pressure and volume of the heart's left ventricle. The prototype device 300 is shown in figs. 17 and 18. The prototype device 300 is described in detail below.

A prototype support member 301 made of a flexible material was provided with electrically conductive lines and patterns 311, 313, 317, in the form of conductor lines 317 on the first side 325 and electrodes 111, 113 on the second side 327 of the prototype support member 301, using standard technology. The dimensions of the prototype support member 301 were: length 35 cm, width 2,1 mm and thickness 50 μm. On the first side 325 of the prototype support member 301 ("inside" of the finished prototype 300) 7 conductor lines 317a-317g were placed/manufactured (fig. 17), 3 of them terminating near a hole 315 over which a pressure sensor chip 303 later was to be mounted (soldered). At the termination (near the hole 315) of each of these 3 lines a soldering pad 318a-318c, in electrical contact with respective line, was placed. The other 4 lines were terminated at via holes 321 connecting the lines to front end electrodes 311a-311d on the second side 327 of the prototype support member 301 ("outside" of the finished device). The via holes 321 connect the lines to the outside electrodes 311, 313 by means of electrically conductive material (via conductors) 323 on the walls of the via holes 321. The front end electrodes 311a-311d were placed with two electrodes on each side of the pressure sensor chip 303. In this embodiment 4 front end electrodes 311 were used but depending on the measurement method or application fewer or more electrodes may be used. The arrangement of the electrodes is adapted to the measurement method or application in question. For the conductor lines 317 and the electrodes 111, 113 the metal copper was used, approximately 20 μm thick. On the first side 325 ("inside") the conductor lines 317 were covered with a layer of a suitable material (for example tin or silver+gold) to enable soldering of the pressure sensor chip 303. On the second side 327 ("outside") the electrodes 111, 113 were covered with a layer of a material suitable (for example gold) to make the device 100 for invasive use suitable to be inserted into the body: That is, to make the device 100 for'invasive use biocompatible or biologically inert.

The conductor lines 317 were 100 μm wide with distances of 100 μm between the lines. The bond pads 303a-303c on the pressure sensor 303 were respectively attached to the bond pads 318a-318c on the prototype support member 301. The pressure sensitive area 303d of the pressure sensor 303 may comprise a pressure sensor membrane 303e (not shown) Solder paste was applied to the bonding pads 318a-318c (fig. 18) by screen printing and the pressure sensor chip 303 was put in place with standard surface mounting electronic production equipment. Subsequently the chip was soldered in a furnace using a suitable temperature curve or profile with a peak temperature of about 230° C.

Having soldered the sensor chip 303 the prototype support member 301 was fed through a funnel-like opening 611 and a through-hole 609 in a tool or jig 600 (figs. 8-10) where the diameter of the through-hole 609 was 0,7 mm. The funnel-like opening 611 was heated in the upper part to 140°C where also a suitable adhesive was provided, in this case PolyCaproLacton (PCL) was used. This adhesive melted and filled the interior of the support member while it was formed to a tube by feeding it through the tool or jig 600. The lower portion of the through-hole 609 was cooled by a Peltier-cooler to enable the adhesive to crystallize. The pressure sensor chip 303 was of a kind adapted to be contained in the small space inside the tube shaped prototype support member 301.

In this way a thin, long rod was formed with a diameter and flexibility suitable for the use as a device for invasive use for insertion through a vein in the neck to reach the right ventricle of the heart or through the aorta to reach the left ventricle of the heart. When the front end of the device is placed in either of the heart's ventricles it can be used to monitor pressure/volume loops. The length of the prototype device 300 needs to be increased to make it suitable for some clinical applications but this can be accomplished with existing techniques for the production of flexible printed wire boards in a way easily derivable for the person skilled in the art.

<u>Further exemplary description of the device 100 for invasive use</u>

It may be advantageous to provide the device 100 for invasive use with a reinforcing or rigidifying element 103 on the inside of the at least partly tube shaped support member 101. The reinforcing or rigidifying element 103 may be provided along the entire length of the device 100 for invasive use or merely along a portion of the length of the device 100 for invasive use.

The reinforcing or rigidifying element 103 may for example comprise a wire or string made of for example metal or polymer, and/or the reinforcing or rigidifying element 103 may comprise the solidified or crystallized adhesive or glue, for example the solidified or crystallized PolyCaproLakton, PLC. The reinforcing or rigidifying element 103 may also comprise a lumen. If the reinforcing or rigidifying element 103 comprises a lumen (or at least one lumen) it may be used for taking samples from inside the body or for distributing substances, like medicine, to the body.

By varying the rigidity of the reinforcing or rigidifying element 103 the rigidity of the device 100 for invasive use can be adapted to different applications, for example the application where the device 100 for invasive use is inserted to the desired location directly through tissue. The device 100 for invasive use may for example be inserted through myocardium and subsequently withdrawn without causing trauma. To vary the rigidity of the reinforcing or rigidifying element 103 may be an advantage since a well adapted degree of rigidity may positively influence the usability of the device 100 for invasive use in a certain application.

To facilitate handling and insertion of the device 100 for invasive use through for instance a catheter guide, the reinforcing or rigidifying element 103 may be double as long as the support member 101 and extend beyond the back end 109 of the device 100 for invasive use (see fig. 3b). After insertion of the device 100 for invasive use the part of the reinforcing or rigidifying element 103 extending beyond the back end 109 may be cut off. The reinforcing or rigidifying element 103 may also extend (*e.g.* about 5-30mm, advantageously about 7-13 mm) beyond the front end 107 of the support member 101 and so replacing or complementing the soft tip 105 (see fig. 1). If the reinforcing or rigidifying element 103 extends beyond the front end 107 of the support member 101 it is advantageous that the extending part of the reinforcing or rigidifying element 103 is bent, for example by using heat, and biocompatible. The length, diameter, rigidity, curvature and other characteristics of the part of the reinforcing or rigidifying element 103 extending beyond the front end 107 of the support member 101 are adapted to the application or use in question. The reinforcing or rigidifying element 103 may in this way enable an effective and precise guidance of the device 100 for invasive use e.g. in a network of blood vessels.

If the device 100 for invasive use is used for monitoring parameters in the ventricle/s of the heart it may be advantageous to provide the device 100 for invasive use with a reinforcing or rigidifying element 103 since the device 100 for invasive use is bent very frequently as a result of the pumping motion of the heart. Without a reinforcing or rigidifying element 103 the device 100 for invasive use may be damaged by the frequent bending. This of course also applies to other applications where the device 100 for invasive use is subjected to frequent bending.

It may also be advantageous to provide the device 100 for invasive use with a soft tip 105 integrated at the front end of the device 100 for invasive use (fig. 7). The tip 105 is advantageously formed when cutting the flexible support member 101 that is used for the device 100 for invasive use. In this way the tip 105 constitutes an integrated part of the device 100 for invasive use. The tip 105 is advantageous when the device 100 for invasive use is inserted into the body, for example when the device 100 is inserted into a ventricle of the heart. Also when the device 100 for invasive use has been inserted and is in its end position the soft tip 105 ensures that the surrounding tissue, muscle or artery or vein is not damaged or penetrated. In one advantageous embodiment the support member 101 is around 50 micrometer thick and the tip 105 is approximately 0.7mm wide, ca 30mm long, and includes a fine graded transition to the approximately 2mm wide part of the support member 101. These measures make the tip 105 soft and prevents that the heart is injured or disturbed (for example arrhythmia) when the device 100 for invasive use e.g. is inserted, withdrawn or in its end position.

It is an advantage that the tip 105 is an integrated part of the device 100 for invasive use, for example the tip 105 can not fall off. In some of the devices for invasive use (often called catheters) known from the background art separate tips of for example platinum have been used and in some cases they have fallen off while the device was inside the body. This is a disadvantage since it can create a dangerous situation for the patient and/or render the examination that is carried out with the device difficult.

It may be advantageous to attach the device 100 for invasive use to another structure, e.g. a medical device such as a feeding probe. When the device 100 for invasive use is attached to a feeding probe nerve signals from the diaphragm may be measured with the at least one front end electrode 111 of the device 100 for invasive use. The attachment may be accomplished by means of for example an adhesive or some other attachment means.

<u>Manufacturing of the device 100 for invasive use described herein</u>

The part of the manufacturing method for the device 100 for invasive use described herein, that relates to the formation of the support member 101 at least partly into a tube shape will now be described more in detail. In fig. 8 the principle of the manufacturing method is shown.

Generally, when manufacturing the device 100 for invasive use, an elongated support member 101 is at least partly brought into a tube shape and the inside of the tube shaped support member 101 is at least partly sealed from the outside. The support member 101 has at least one electrical conductor on one or both sides of the support member 101 and may advantageously be equipped with at least one electronic component and/or at least one microelectromechanical system 200. It may be an advantage to mount the at least one electronic component and/or at least one microelectromechanical system 200 on the inside of the at least partly tube shaped support member 101 but at least one component or system may also be mounted on the outside of the a least partly tube shaped support member 101, especially if it is integrated in the support member 101. Advantageously, the at least one electronic component and/or at least one microelectromechanical system 200 is mounted on the support member 101 before the support member 101 is, at least partly, formed into a tube shape.

In one embodiment of the manufacturing method described herein the device 100 for invasive use is equipped with a reinforcing or rigidifying element 103 on the inside of the at least partly tube shaped support member 101.

In this embodiment the support member 101 is provided with a tip 105 at at least one of the ends of the support member. The tip 105 is narrower than the rest of the support member and may have a length of approximately 10 to 50 mm, preferably 15 to 30 mm. When forming the support member 101 at least partly into a tube shape, a jig or tool 600 made out of a block of material like metal or plastic is used. The metal used may for example be steel, brass, copper or any other alloy. A suitable plastic may for example be polymethacrylate, known as Plexiglass™.

The jig or tool 600 is provided with a small hole 609 having a funnel-like opening 611. The hole 609 and the funnel-like opening 611 are adapted not to damage the support member 101 or the conductive patterns 111, 113 or other elements provided on the second side 127 of the support member 101. For example may a lining be provided in the hole 609 and/or funnel-like opening 611.

The tip 105 of the support member 101 is.threaded trough the funnel-like opening 611 and the small hole 609. The opening 611 is filled with an adhesive or glue 601, it may be advantageous to use PolyCaproLacton (PCL) which has a good adhesion to polyimide. The adhesive or glue 601 may be distributed by means of a dispenser. Generally, an adhesive 601 is selected that has a good adhesion to the material of the support member 101. The adhesion between the adhesive 601 and the support member 101 needs to be good to maintain the support member 101 in a tube shape. The adhesive 601 is melted and fills the support member 101. When the support member 101 is pulled through the lower part of the hole, it is cooled and the PCL crystallizes (it becomes solid) and forms a reinforcing or rigidifying element 103. The reinforcing or rigidifying element 103 may comprise the solidified adhesive material, a separate reinforcing or rigidifying element or a combination of the solidified adhesive material and the separate reinforcing or rigidifying element. The separate reinforcing or rigidifying element, e.g. a wire or the like, is placed on the inside of the support member 101. The principle of how the support member 101 is formed is shown in figs. 8 and 9. If there are via holes 121 in the support member these will filled with adhesive material as the support member 101 is being fed through the tool 600. The adhesive material will fill the via holes 121 completely and will substantially be in line with the outside surface of the support member 101. If the device 100 for invasive use is not covered with a biocompatible material, like a biocompatible hydrogel, it is advantageous that the adhesive material used is biocompatible.

Now the manufacturing steps will be described more in detail. The jig or tool 600 comprises three layers, an upper heating layer 603, a middle insulation layer 605 and a bottom cooling layer 607. A hole 609 with a diameter of 0.7mm passes through the three layers in the jig or tool 600. The upper layer 603 is heated to a temperature between +75 and +200°C and the lower layer 607 is cooled to a temperature between -10 and +25°C. A higher heating temperature requires a lower cooling temperature, but it may be advantageous to use a temperature of +140°C in the upper layer 603 and +5°C in the lower layer 607. The middle layer 605 is used as insulation layer.

The support member 101 is 2mm wide and at least one of the ends of the support member 101 is provided with a 0.7mm wide tip 105. One of the tips is, in an advantageous embodiment, ca 30 mm long, and includes a fine graded transition to the 2mm wide part of the support member 101. That tip 105 is pulled down into the funnel-like opening 611, which has a maximum diameter of 7.9 mm and connects to the hole 609 having a diameter of 0.7 mm. It may be advantageous to use a steel wire loop that is threaded with the tip 105 of the support member 101 to pull down the support member 101 through the hole 609.

Advantageously the width of the support member 101 substantially corresponds to the diameter of the hole 609 times $\pi$. In this way the support member 101 is formed into a tube without any overlap so that one of the longer edges of the support member 101 abuts or faces the other longer edge of the support member 101.

The funnel-like opening 611 is shaped as shown in figs. 9 and 10. One side of the funnel-like opening 611 is substantially vertically leading down to the hole 609 and the inclination-angle of the funnel-like opening 611 gets gradually smaller as one moves from the substantially vertical side towards the opposite side of the funnel-like opening 611 where the inclination-angle is approximately 40-70 degrees, advantageously 60 degrees. The hole 609 may be lined, e.g. with a lining tube 613 as shown in fig. 10. The lining of the hole 609 is advantageous since it is a convenient way to ensure that the hole 609 has a smooth surface that will not damage the support member 101. The tube 613 extends a certain distance (in fig. 10 2,2mm) passed the joint between the layers 603 and 605 to ensure that no adhesive or glue 601 enters the joint between the layers 603 and 605. In fig. 10 the lining tube 613 has an outer diameter of 2mm, which of course is just an example and may be varied depending on the circumstances. In fig. 10 the figuring 0,7mm denotes the diameter of the support member 101.

The front part of the support member 101 is placed with the side that will later be the outside of the device 100 for invasive use in contact with the substantially vertical side of the funnel-like opening 611. The rest of the support member 101 is leaned backwards from the funnel-like opening 611 with an angle of about 40-60°. This is done by attaching a thread to the free end of the support member 101 and letting it rest on a stick fastened a few decimetres diagonally behind the jig or tool 600.

When the tip 105 is pulled, the support member 101 is folded to fit into the hole 609 and the tip 105 is pulled until a length of approximately 5-10mm of the 2mm wide folded support member 101 has exited the hole 609.

By letting the support member 101 lean backwards it is forced to stay close to the approximately vertical side of the funnel-like opening 611 to prevent adhesive or glue 601 from flowing down into the hole 609 on the outside of the support member 101. If adhesive 601 would attach to the outside of the support member 101 that could interfere with the access hole 115 and the membrane 131 that may be placed in the access hole 115. The leaning also makes the support member 101 automatically fold with the side, which later will be the inner wall of the catheter, on its inside.

Four to five PCL-pellets are placed in the opening of the funnel-like opening 611 and while the pellets melt, the piece of support member 101 coming out through the hole 609 in the jig or tool 600 is attached to a clip (not shown). The clip is coupled to a pulling mechanism by a thread, the pulling mechanism may for example be driven by a motor such as a DC-motor.

When the PCL is melted, it becomes transparent and soft. Then the pulling mechanism is activated or started and pulls the support member 101 through the hole 609 at a speed of up to about 7cm/min or faster but advantageously 1-5 cm/min and more advantageously about 2.8cm/min. The speed depends of the heating and cooling temperatures. That is because the pulling speed has to be slow enough to let the melted PCL flow into the support member 101 and also to let the PCL crystallize while passing the cooling part of the hole 609.

To prevent the clip from turning around and giving a spiral shaped joint to the support member 101, means are provided to prevent the clip from turning. For example, the clip may be held in place with a stiff board, which the clip leans towards as it moves downwards.

When the whole support member 101 has passed through the hole 609, the support member 101 is caught by hand and released from the clip. It may in some cases be desirable not to form the last part of the support member 101 into a tube shape. In this case the supply of adhesive 601 is simply stopped when the end of the support member 101 is approaching and the last part of the support member 101 is drawn through the jig or tool 600 without supply of adhesive 601.

It may also be possible to pull the support member 101 through the tool or jig 600 at an angle so that the joint between the edges of the support member 101 forms a spiral so that the edges of the support member 101 overlap one another to provide a more rigid device 100 for invasive use. It may also be possible to make the spiral shape so that there is no overlap of the side edges of the support member 101. In this way, glue or adhesive 601 may be applied to the inside surface close to the side edges to form the support member 101 into a tube shape that is more rigid than when the joint between the side edges is straight.

An alternative to filling the at least partly tube shaped support member 101 with glue or adhesive 601 may be to feed a meltable string of solid glue or adhesive or another suitable polymer/additive with appropriate diameter through the funnel-shaped opening 611 and hole 609 simultaneously with the support member 101. A layer or primer may be applied to the inside of the support member 101 to enhance the adhesion between the meltable string and the inner surface of the support member 101 during the roll-up process. The layer may also protect the at least one electronic component and/or at least one microelectromechanical system 200 from the glue or adhesive 601 if necessary. Examples of such layers are Polycaprolacton lacquer and Parylene. A non-limiting example of Parylene is the product Parylene HT™ (Specialty Coating Systems, Indianapolis, USA). The meltable string may melt (partly, only on the surface, or fully) and fill the support member 101 in the upper end and be solidified in the lower end as described above. Any other method of at least partly forming a tube of the support member 101 may also be used, even though the method described here may be advantageous.

It is also possible to use an adhesive that can be solidified by means of UV-radiation.

It is as well possible to use welding, for example laser welding, to weld the adjacent edges of the support member 101 to each other. In this case the jig or tool 600 may be provided with welding equipment that welds the edges of the support member 101 to each other as the support member 101 is drawn through the jig or tool 600. In this case the support member 101 may also be provided with a separate reinforcing or rigidifying element 103 as the support member 101 is drawn through the jig or tool 600. The reinforcing or rigidifying element 103 may advantageously be provided on the inside of the at least partly tube shaped support member 101.

The reinforcing or rigidifying element 103 may be attached to the clip together with the tip 105. The reinforcing or rigidifying element 103 may comprise at least one optical fibre or waveguide 129 for providing a communication possibility between the back end electrodes 113 and the at least one electronic component and/or at least one microelectromechanical system 200 and/or at least one front end electrode 111 of the device 100 for invasive use. The at least one optical fibre or waveguide may be used in addition to, or instead of, the electrically conductive lines or patterns 117.

When using welding to join the adjacent edges of the support member 101 to each other, so as to at least partly form the support member 101 into a tube shape, the front end of the support member 101 may be sealed with an adhesive, by means of the reinforcing or rigidifying element 103, or by means of a plug of a suitable material.

It is possible to produce devices 100 for invasive use in great numbers efficiently. The support members 101 may be manufactured simultaneously in great numbers. In one example, support members 101 are manufactured from sheets or panels 133 of a suitable material as shown in Fig. 11. Common widths of the panels or sheets 133 are 30 or 45cm, which allows hundreds of support members 101 (approximately 1-2 mm wide) to be manufactured simultaneously. The support members 101 are separated by perforations (done for example by milling or laser ablation) to make it easy to separate them. This allows simultaneous formation of several devices 100 for invasive use as depicted in Fig. 12. Water may be used to cool the bottom part or cooling layer 607 of the tool or jig 600. It is also possible to make the production continuous as indicated in Fig. 13. The support members 101 are preferably separated from one another by a suitable perforation or other suitable technologies. The perforation may be added before the perforated sheet or panel 133 enter the tool or jig 600. Here two standard methods are combined with the device 100 for invasive use described herein in a continuous production process. First, the support members 101 are subjected to standard process steps used today by manufacturers of flexible printed wire boards, such as via drilling, pattern formation by lithography and etching. Conductors may also be formed by ink jet printing or in other ways. Next, the at least one electronic component and/or at least one microelectromechanical system 200 is attached by standard pick-and-place equipment using conducing glue, soldering or some other method. Finally, the sheet or panel 133 is fed into a tool or jig 600 with several parallel holes 609 with funnels-shaped openings 611. The feeding mechanism is omitted in the figure. This would constitute a fully continuous process. Devices 100 for invasive use can be cut off in batches after passage of the tool or jig 600.

Hence, the device 100 for invasive use described herein is manufactured with proven methods used in electronics production, apart from the formation of the support member 101 into a tube shape, or at least partly into a tube shape. However, as described above, this latter step can be performed so as to give the finished device 100 excellent mechanical properties and where the device 100 has a construction of low complexity. This gives the device 100 high reliability and it can be manufactured cost effective and still have outstanding electrical and mechanical properties.

One advantage with the device for invasive use described herein is that the construction is relatively simple. Thereby reliability can be improved. Basically the support member (101) itself constitutes a device suitable for invasive use. Since the construction is relatively simple the device 100 for invasive use may also be manufactured relatively inexpensive which facilitates the use of the device 100 for invasive use as a single use article.

The manufacturing process brings advantages for example in terms of automation. The manufacturing process is also easy to implement in a bigger scale since several devices can be manufactured in parallel.

It may also be advantageous to provide the support member 101 with a sharp point for facilitating the insertion of the device 100 for invasive use through tissue to an organ. The sharp point may be in the form of a needle.

It may also be advantageous that the device 100 for invasive use is adapted to extend from an organ at least to a point of surgical incision.

It may furthermore be advantageous that the device 100 for invasive use is adapted to extend from the heart at least to the groin.

System

The device 100 for invasive use may be used as part of a system for monitoring, examining or treating a patient. As an example of such a system, a system 700 for monitoring the heart will be described (see in particular figs. 3, 19-25). The system 700 enables the function of the left ventricle of the heart to be monitored in real time, during for example invasive heart examination, heart operation or post operative treatment. The system 700 comprises the device 100 for invasive use, an accompanying contact (not shown) and equipment for signal processing and displaying. A device 100 for invasive use, which is thin, supple and comprises a soft tip 105, is introduced or inserted through an artery in the groin so that the front end 107 of the device 100 for invasive use is placed in the left ventricle of the heart. A catheter guide is advantageously used when the device 100 for invasive use is introduced.

Advantageously the device 100 for invasive use may be inserted into the femoral aorta and pushed through the aorta and into the left ventricle of the heart. With the device 100 for invasive use volume and pressure in the left ventricle of the heart can be measured and a Pressure- Volume diagram can be displayed. A physician can in this Pressure- Volume diagram read or derive parameters for the function of the heart and diagnose illness or malfunction. The device 100 for invasive use is in this case implemented as a multi-functional device combining means for conductance/impedance measurement (front end electrodes 111) with a pressure sensor 201.

The volume measurement functions as an impedance measurement. On the device 100 for invasive use, four front end electrodes 111a-111d are placed, two of them excitation electrodes 111a, 111d and between these, two measurement electrodes 111b, 111c. The device 100 for invasive use is placed so that the fore part is substantially aligned with the longitudinal axis of the left ventricle. The excitation electrodes 111a, 111d are placed to be on a level with the apex of the heart (apex *cordis)* and the base of the heart *(basis cordis).* The two excitation electrodes 111a, 111d are fed with a voltage $U_{excitation}$ so that an alternating current $I_{excitation}$ with the frequency 20 kHz and the intensity 100 micro-ampere flows between the two excitation electrodes, this is in accordance with the standard IEC-601. Advantageously alternating current is used to avoid interference with cardiac electro-physiology. An electromagnetical field will be generated in the left ventricle, which creates a voltage $U_{measured}$ that may be measured with the two measurement electrodes 111b, 111c. The measured voltage $U_{measured}$ will be proportional to the impedance $Z_{meas.elec.}$ between the two measurement electrodes 111b, 111c according to the equation:

$$U_{measured}=Z_{meas.elec.}*I_{excitation} \quad (1).$$

The impedance $Z_{meas.elec.}$ will in turn be dependent on the volume in the ventricle. The volume can be calculated from the equation:

$$V=\rho*L^2/G \quad (2)$$

where p denotes the resistivity of the blood, L denotes the distance between the two measurement electrodes 111b and 111c, and G denotes the measured conductance, which is the real part of the inverted value of the impedance $Z_{meas.elec.}$. The measured voltage $U_{measured}$ is amplified, filtered, demodulated, used to compute the volume V using equations (1) and (2), and the signal is then subjected to an analog-to-digital (A/D) conversion so that it conveniently can be presented graphically. The demodulation follows the principle of phase sensitive rectifier which means that the phase of the AC signal $U_{measured}$ is compared to $U_{excitaion}$ and the signal Umeasured is converted into two DC-voltage levels where one correspond to the real valued voltage over the impedance and the other to the imaginary valued part. In this case the impedance is the impedance present between the two measurement electrodes. The demodulation works as follows. The signal $U_{measured}$ is divided into two signals where one is phase shifted 180°. Both signals $U_{measured}$ and Umeasured (+180°) are then switched in two switches, switch_0 and switch_90. Each of the two switches are controlled by a control pulse, where one control pulse (CP_0) has the same phase as the signal $U_{excitation}$ and the other (CP_90) is phase shifted +90° in relation to $U_{excitation}$. Switch_0 is controlled by CP_0 and switch_90 is controlled by CP_90.

Both switches have both the signals $U_{measured}$ and Umeasured (+180°) as inputs. The switches will then let $U_{measured}$ pass when the control pulse is high (logic 1), and let $U_{measured}$ (+180°) pass when the control pulse is low (logic 0). For both of these output signals from the switches ($U_{measured_SW_0}$ and $U_{measured_SW_90}$) the mean value is computed ($U_{measured_SW_0_mean}$ and $U_{measured_SN_90_mean}$). These mean value levels will correspond proportionally to the real and imaginary valued voltages over the impedance in the left ventricle. The mean value of the output signal.from switch_0 ($U_{measured_SW_0_mean}$) will correspond to the real valued voltage, the mean value of the output signal from switch_ 90 ($U_{measured_sw_90_mean}$) will correspond to the imaginary valued voltage. With $U_{measured_SW_0_mean}$ and

$I_{excitation}$ the conductance G can be computed using eq. (1) and inverting the result and hence also the volume V according to equation (2) can be computed.

In fig. 23a the input signals for switch_0 are shown. Reference sign 23a1 denotes the control pulse CP_0 to switch_0, 23a2 denotes $U_{measured}$, 23a3 denotes $U_{measured}$ (+180). The measured signal $U_{measured}$ has the same phase as the control pulse CP_0 which corresponds to a real valued impedance.

In fig. 23b the input signals for switch_90 are shown. Reference sign 23b1 denotes the control pulse CP_90 to switch_90, 23b2 denotes $U_{measured}$, 23b3 denotes $U_{measured}$ (+180).

In fig. 24 the outputs from the switches switch_0 and switch_90 are shown ($U_{measured_SW_0}$ and $U_{measured_SW_90}$) together with their respective mean values ($U_{measured_SW_0_mean}$ and $U_{measured_SW_90_mean}$).

Both of these signals ($U_{measured_SW_0_mean}$ and $U_{measured_SW_90_mean}$) are then subjected to an analog-to-digital (A/D) conversion. In fig. 25 the circuit diagram of the switches is shown.

The system works well as an impedance meter and measures the impedance with good accuracy approximately +0.5 ohm. The measurement of the volume is however an approximation and is dependent on that the system is adjusted for a specific positioning of the device 100 for invasive use. Optimally the fore part of the device 100 for invasive use will be placed along the vertical axis of the left ventricle. The accuracy of the volume measurement will be approximately $\pm$3 ml, but be better for lower volumes (under 120 ml). The impedance of the blood is much lower than that of the surrounding tissues which is vital for the measurement to work. However, the surrounding tissues will give a contribution to the measured impedance which will have to be compensated for. By injecting a well known volume of saline solution and measure the change in impedance, the contribution to the impedance from the surrounding tissues can be calculated. Another method is to calibrate the system to the patient's volume measured by other measurements, for example the measurement of stroke volume done with thermo dilution or ultrasound.

For the pressure measurement a pressure sensor 201 is used. The pressure sensor 201 is mounted on the device 100 for invasive use between the two measurement electrodes 111b and 111c that is used for the volume measurement. The pressure sensor 201 is a MEMS-chip (Micro Electro Mechanical Systems) that in this embodiment comprises two resistors, $R_p$ and $R_t$. When the pressure exerted on the pressure sensor 201 changes, the resistance for one of the resistors ($R_p$) changes in proportion to the change in pressure. The pressure sensor 201 contains a half bridge that is connected to two other resistors $R_1$ and $R_2$ to form a complete Wheatstone bridge. The resistors $R_1$ and $R_2$ may for example be incorporated in the unit for Amplification or Filtering or they may be placed on the device 100 for invasive use. Figs. 3 and 17 show the embodiment where the resistors $R_1$ and $R_2$ are placed outside the device 100 for invasive use. A voltage (E in fig. 20) of the direct current type, a DC voltage, of approximately 1-2 Volts is connected to the Wheatstone bridge and the output signal $U_{pressure}$ from the Wheatstone bridge will be proportional to the pressure exerted on the pressure sensor 201. The sensitivity of the pressure measurement depends on the type of pressure sensor 201 used. For a number of common pressure sensors the sensitivity may vary from 28.6 $\mu$V/V/kPa to 102,1 $\mu$V/V/kPa. Four pressure sensors based on the same principle have been used for evaluation. The sensitivity of the pressure sensors is dependent on their geometry. The larger the sensor is, the larger is its pressure sensitive membrane and therefore its sensitivity.

The output signal $U_{pressure}$ from the pressure sensor 201 is amplified, filtered and subjected to an analog-to-digital (A/D) conversion so that it conveniently can be presented graphically (see fig. 19). Varying values of the components in the pressure sensor 201, for example varying resistance values of the resistors, will cause imbalance in the Wheatstone bridge. This imbalance can not be neglected but has to be compensated for, such an imbalance may influence the measured/computed pressure value with as much as +/- 20 kPa.

Since the pressure sensor 201 is not ideal, calibration data for each device 100 for invasive use are saved in a memory placed in a contact (not shown) that is used to connect the device 100 for invasive use to the equipment for signal processing and displaying. Each device 100 for invasive use is provided with a dedicated contact containing calibration data for that particular device 100 for invasive use. The calibration data will include the sensitivity of the pressure sensor 201 and its contribution to the imbalance in the Wheatstone bridge. It is advantageous to calibrate the system to the atmospheric pressure prevailing when the device 100 for invasive use is used. When calibrated the accuracy for the pressure measurement will be good, approximately +/- 133 Pa. An accuracy in this range is advantageous for the application of monitoring heart function.

In figs. 20-22 $R_p$ is the pressure sensitive resistor. $R_t$ is a reference resistor with the same temperature dependence as $R_p$ and hence has the function of compensating for temperature. The pressure sensor 201 is connected to the resistors $R_1$ and $R_2$ and to ground by means of three connectors, for example bond pads, 201a-201c. The numbers 1, 2 and 3 denotes the connection points for the connectors 201a-201c (figs. 6, 20-22). The frame around the resistor $R_p$ in fig. 22 symbolises the pressure sensitive area 201d (which may include a pressure sensor membrane 201e) on the pressure sensor 201.

Also the use of the device 100 for invasive use brings advantages since several examinations relating to invasive use can be done more easily and/or to a lower cost, and also examinations that have not been able to perform previously can now be performed with the device 100 for invasive use described herein.

Although particular embodiments have been disclosed herein in detail, this has been done by way of example for purposes of illustration only, and is not intended to be limiting with respect to the scope of the appended claims that follow. In particular, it is contemplated by the inventor that various substitutions, alterations, and modifications may be made to the invention without departing from the scope of the invention as defined by the claims.

*Reference signs*

Device for invasive use - 100
support member (e.g. foil) - 101
Reinforcing or rigidifying element (wire, solidified glue, optical fibre or the like) - 103
Tip (elongated, thin) of support member - 105
Front end of support member - 107
Back end of support member - 109
Front end electrodes - 111
Back end electrodes - 113
Access hole in support member, for pressure sensor - 115
Conductive lines or patterns on the first side, inside of the support member - 117
Soldering or bond pad on support member - 118
Via holes - 121
Via conductors - 123
First side of the support member 101 - 125
Second side of the support member 101 - 127
Optical fibre or waveguide - 129
Support member'membrane - 131
Sheet or panel of material suitable for the support member - 133
"Non-functional" or "dummy" part - 135
Perforation for the "non-functional" part - 137

electronic component or microelectromechanical system - 200
Pressure sensor - 201
Bond pads on pressure sensor - 201a-201c
Pressure sensitive area on pressure sensor - 201d
Pressure sensor membrane - 201e
Via holes on pressure sensor - 203a-203c
Via conductors in via holes on pressure sensor - 205a-205c
Electrical conductors on underside of pressure sensor - 207
Bonding wires connecting bond pads 118a-118c on support member with bond pads 201a-201c on pressure sensor - 209a-209c

Prototype - 300
Prototype support member - 301
Pressure sensor on prototype - 303
Front end of prototype support member - 307
Back end of prototype support member - 309
Hole in prototype support member, for pressure sensor - 315
Conductive lines or patterns prototype support member - 317
Bond pads on prototype support member, for pressure sensor - 318a-318c
Via conductors on prototype support member - 323
First side of the prototype support member 301 - 325
Second side of the prototype support member 301 - 327

Tool or jig - 600
Adhesive or glue - 601
Heating layer of tool or jig - 603
Insulation layer of tool or jig - 605
Cooling layer of tool or jig - 607
Through hole through hole or jig - 609
Funnel-shaped opening in tool or jig - 611
Lining tube for tool 600 - 613

System - 700
Signals in the system - 26a1-26a3, 26b1-26b3

## Claims

**1.** A device (100) for invasive use, comprising a support member (101) comprising a flexible material, wherein:

a. the support member (101) comprises at least one layer of at least one electrically conductive line or pattern (117) thereon;
b. the support member (101) is at least partly formed into an elongated tube, and the inside of the support member (101) is at least partly sealed from the outside of the support member (101);
c. at least one electrically conductive line or pattern (117) of said at least one layer extends on the inside of the elongated tube (101); and
d. the support member (101) comprises at least one sensing, stimulating and/or processing element (111, 113, 200, 201), and wherein

the support member (101) has at least one opening (115) through the longitudinal wall of the elongated tube and at least one of the at least one sensing, stimulating and/or processing element (200, 201) is aligned with said at least one opening (115).

**2.** A device (100) for invasive use according to claim 1, wherein the support member (101) is at least partly filled with a flexible resilient material, such as an adhesive or a polymer (601).

**3.** A device (100) for invasive use according to claim 1, wherein the support member (101) is completely filled with a flexible resilient material, such as an adhesive or a polymer (601).

**4.** A device (100) for invasive use according to claim 1, wherein the inside of the support member (101) is completely sealed from the outside of the support member (101).

**5.** A device (100) for invasive use according to claim 1, wherein the device (100) for invasive use is adapted to be provisionally located in a body by surgical invasion for monitoring or influencing the function of an organ.

**6.** A device (100) for invasive use according to claim 5, wherein the device (100) for invasive use is adapted to be provisionally located in a body by surgical invasion for monitoring or influencing the function of a heart.

**7.** A device (100) for invasive use according to claim 1, wherein the adjacent edges of the support member are at least partly joined by welding.

**8.** A device (100) for invasive use according to claim 1, wherein the adjacent edges of the support member (101) are at least partly joined by an adhesive.

**9.** A device (100) for invasive use according to claim 1, wherein the at least one sensing, stimulating and/or processing element comprises at least one electronic component or microelectromechanical system (200), provided on the inside of the at least partly tube shaped support member (101).

**10.** A device (100) for invasive use according to claim 9, wherein the at least one electronic component or microelectromechanical system (200, 201) is chosen among a pressure sensor, a voltage sensor, a pH sensor, a temperature sensor, a gas sensor, a component for detecting or quantifying a reagent, and a drug delivery device.

**11.** A device (100) for invasive use according to claim 1, wherein at least one electrode (111, 113) is placed on the outside of the elongated tube (101).

**12.** A device (100) for invasive use according to claim 1, wherein at least one electrode (111, 113) is placed on the inside of the elongated tube (101).

**13.** A device (100) for invasive use according to claim 11 or 12 wherein at least four electrodes (111, 113) are provided, said at least four electrodes constituting a volume sensor.

**14.** A device (100) for invasive use according to 30 claim 1 wherein there is provided at least one reinforcing or rigidifying element (103) on the inside of the support member (101).

**15.** A device (100) for invasive use according to claim 14 wherein the at least one reinforcing or rigidifying element (103) extends beyond one or both ends of the support member (101).

**16.** A device (100) for invasive use according to claim 14 or 15 wherein the at least one reinforcing or rigidifying element (103) comprises an optical fibre or waveguide (129).

**17.** A device (100) for invasive use according to any one of the claims 9 or 10 wherein there is provided at least one optical fibre or waveguide (129) on the inside of the support member 101.

**18.** A device (100) for invasive use according to any one of the claims 9, 10 or 17 wherein the at least one electronic component or microelectromechanical system (200, 201) is placed adjacent a front end (107) of the support member (101) and in operational contact with the at least one electrically conductive line or pattern (117) or said at least one optical fibre or waveguide (129).

**Patentansprüche**

**1.** Vorrichtung (100) zur invasiven Verwendung, die ein Trägerelement (101) umfasst, das ein flexibles Material enthält, wobei:

a. das Trägerelement (101) mindestens eine Schicht aus mindestens einer elektrisch leitfähigen Leitung oder mindestens einem elektrisch leitfähigen Muster (117) darauf umfasst;
b. das Trägerelement (101) zumindest teilweise als eine längliche Röhre geformt ist, und das Innere des Trägerelements (101) zumindest teilweise gegenüber dem Äußeren des Trägerelements (101) abgedichtet ist;
c. mindestens eine elektrisch leitfähige Leitung oder mindestens ein elektrisch leitfähiges Muster (117) der mindestens einen Schicht auf der Innenseite der länglichen Röhre (101) verläuft; und
d. das Trägerelement (101) mindestens ein Erfassungs-, Stimulations- und/oder Verarbeitungselement (111, 113, 200, 201) umfasst, und wobei

das Trägerelement (101) mindestens eine Öffnung (115) durch die Längswand der länglichen Röhre aufweist und mindestens eins von dem mindestens einen Erfassungs-, Stimulations- und/oder Verarbeitungselement (220, 201) nach der mindestens einen Öffnung (115) ausgerichtet ist.

2. Vorrichtung (100) zur invasiven Verwendung nach Anspruch 1, wobei das Trägerelement (101) zumindest teilweise mit einem flexiblen elastischen Material wie einem Klebstoff oder einem Polymer (601) gefüllt ist.

3. Vorrichtung (100) zur invasiven Verwendung nach Anspruch 1, wobei das Trägerelement (101) vollständig mit einem flexiblen elastischen Material wie einem Klebstoff oder einem Polymer (601) gefüllt ist.

4. Vorrichtung (100) zur invasiven Verwendung nach Anspruch 1, wobei das Innere des Trägerelements (101) gegenüber dem Äußeren des Trägerelements (101) vollständig abgedichtet ist.

5. Vorrichtung (100) zur invasiven Verwendung nach Anspruch 1, wobei die Vorrichtung (100) zur invasiven Verwendung angepasst ist, mittels chirurgischen Eingriffs provisorisch in einem Körper angeordnet zu werden, um die Funktion eines Organs zu überwachen oder zu beeinflussen.

6. Vorrichtung (100) zur invasiven Verwendung nach Anspruch 5, wobei die Vorrichtung (100) zur invasiven Verwendung angepasst ist, mittels chirurgischen Eingriffs provisorisch in einem Körper angeordnet zu werden, um die Funktion eines Herzens zu überwachen oder zu beeinflussen.

7. Vorrichtung (100) zur invasiven Verwendung nach Anspruch 1, wobei die benachbarten Ränder des Trägerelements zumindest teilweise durch Schweißen verbunden sind.

8. Vorrichtung (100) zur invasiven Verwendung nach Anspruch 1, wobei die benachbarten Ränder des Trägerelements (101) zumindest teilweise durch einen Klebstoff verbunden sind.

9. Vorrichtung (100) zur invasiven Verwendung nach Anspruch 1, wobei das mindestens eine Erfassungs-, Stimulations- und/oder Verarbeitungselement mindestens ein elektronisches Bauteil oder mikroelektromechanisches System (200) umfasst, das an der Innenseite des zumindest teilweise röhrenförmigen Trägerelements (101) vorgesehen ist.

10. Vorrichtung (100) zur invasiven Verwendung nach Anspruch 9, wobei das mindestens eine elektronische Bauteil oder mikroelektromechanische System (200, 201) aus einem Drucksensor, einem Spannungssensor, einem pH-Sensor, einem Temperatursensor, einem Gassensor, einem Bauteil zum Nachweisen oder mengenmäßigen Bestimmen eines Reagens und einer Medikamentenabgabevorrichtung ausgewählt ist.

11. Vorrichtung (100) zur invasiven Verwendung nach Anspruch 1, wobei mindestens eine Elektrode (111, 113) an der Außenseite der länglichen Röhre (101) platziert ist.

12. Vorrichtung (100) zur invasiven Verwendung nach Anspruch 1, wobei mindestens eine Elektrode (111, 113) an der Innenseite der länglichen Röhre (101) platziert ist.

13. Vorrichtung (100) zur invasiven Verwendung nach Anspruch 11 oder 12, wobei mindestens vier Elektroden (111, 113) vorgesehen sind, wobei die mindestens vier Elektroden einen Volumensensor darstellen.

14. Vorrichtung (100) zur invasiven Verwendung nach Anspruch 1, wobei mindestens ein Verstärkungs- oder Versteifungselement (103) an der Innenseite des Trägerelements (101) vorgesehen ist.

15. Vorrichtung (100) zur invasiven Verwendung nach Anspruch 14, wobei das mindestens eine Verstärkungs- oder Versteifungselement (103) über ein Ende oder beide Enden des Trägerelements (101) hinausgeht.

16. Vorrichtung (100) zur invasiven Verwendung nach Anspruch 14 oder 15, wobei das mindestens eine Verstärkungs- oder Versteifungselement (103) eine Glasfaser oder einen Wellenleiter (129) umfasst.

17. Vorrichtung (100) zur invasiven Verwendung nach einem der Ansprüche 9 oder 10, wobei mindestens eine Glasfaser oder mindestens ein Wellenleiter (129) an der Innenseite des Trägerelements 101 vorgesehen ist.

18. Vorrichtung (100) zur invasiven Verwendung nach einem der Ansprüche 9, 10 oder 17, wobei das mindestens eine elektronische Bauteil oder mikroelektromechanische System (200, 201) angrenzend an ein vorderes Ende (107) des Trägerelements (101) und in funktionsmäßigem Kontakt mit der mindestens einen elektrisch leitfähigen Leitung oder dem mindestens einen elektrisch leitfähigen Muster (117) oder der mindestens einen Glasfaser oder dem mindestens einen Wellenleiter (129) platziert ist.

## Revendications

1. Dispositif (100) pour utilisation invasive, comprenant un élément de support (101) comprenant un matériau flexible, dans lequel :

a. l'élément de support (101) comprend au moins une couche d'au moins une ligne ou impression conductrice électriquement (117) sur celui-ci ;
b. l'élément de support (101) est au moins partiellement formé en un tube allongé et l'intérieur de l'élément de support (101) est au moins partiellement étanche par rapport à l'extérieur de l'élément de support (101);
c. au moins une ligne ou impression conductrice électriquement (117) de ladite au moins une couche s'étend sur l'intérieur du tube allongé (101); et
d. l'élément de support (101) comprend au moins un élément de détection, de stimulation et/ou de traitement (111, 113, 200, 201), et dans lequel

l'élément de support (101) présente au moins une ouverture (115) à travers la paroi longitudinale du tube allongé et au moins un du au moins un élément de détection, de stimulation et/ou de traitement (200, 201) est aligné avec ladite au moins une ouverture (115).

2. Dispositif (100) pour utilisation invasive selon la revendication 1, dans lequel l'élément de support (101) est au moins partiellement rempli avec un matériau résilient flexible, tel qu'un adhésif ou un polymère (601).

3. Dispositif (100) pour utilisation invasive selon la revendication 1, dans lequel l'élément de support (101) est complètement rempli avec un matériau résilient flexible, tel qu'un adhésif ou un polymère (601).

4. Dispositif (100) pour utilisation invasive selon la revendication 1, dans lequel l'intérieur de l'élément de support (101) est complètement étanche par rapport à l'extérieur de l'élément de support (101).

5. Dispositif (100) pour utilisation invasive selon la revendication 1, dans lequel le dispositif (100) pour utilisation invasive est adapté pour être situé provisoirement dans un corps par intervention chirurgicale invasive pour surveiller ou influencer la fonction d'un organe.

6. Dispositif (100) pour utilisation invasive selon la revendication 5, dans lequel le dispositif (100) pour utilisation invasive est adapté pour être situé provisoirement dans un corps par intervention chirurgicale invasive pour surveiller ou influencer la fonction d'un coeur.

7. Dispositif (100) pour utilisation invasive selon la revendication 1, dans lequel les bords adjacents de l'élément de support sont au moins partiellement joints par soudage.

8. Dispositif (100) pour utilisation invasive selon la revendication 1, dans lequel les bords adjacents de l'élément de support (101) sont au moins partiellement joints par un adhésif.

9. Dispositif (100) pour utilisation invasive selon la revendication 1, dans lequel le au moins un élément de détection, de stimulation et/ou de traitement comprend au moins un composant électronique ou système microélectromécanique (200), prévu sur l'intérieur de l'élément de support (101) au moins partiellement en forme de tube.

10. Dispositif (100) pour utilisation invasive selon la revendication 9, dans lequel le au moins un composant électronique ou système microélectromécanique (200, 201) est choisi parmi un capteur de pression, un capteur de tension, un capteur de pH, un capteur de température, un capteur de gaz, un composant pour détecter ou quantifier un réactif, et un dispositif d'administration de médicament.

11. Dispositif (100) pour utilisation invasive selon la revendication 1, dans lequel au moins une électrode (111, 113) est placée sur l'extérieur du tube allongé (101).

12. Dispositif (100) pour utilisation invasive selon la revendication 1, dans lequel au moins une électrode (111, 113) est placée sur l'intérieur du tube allongé (101).

13. Dispositif (100) pour utilisation invasive selon la revendication 11 ou 12, dans lequel au moins quatre électrodes (111, 113) sont prévues, lesdites au moins quatre électrodes constituant un capteur de volume.

14. Dispositif (100) pour utilisation invasive selon la revendication 1, dans lequel est prévu au moins un élément de renforcement ou de rigidification (103) sur l'intérieur de l'élément de support (101).

15. Dispositif (100) pour utilisation invasive selon la revendication 14, dans lequel le au moins un élément de renforcement ou de rigidification (103) s'étend au delà d'une ou des deux extrémités de l'élément de support (101).

16. Dispositif (100) pour utilisation invasive selon la revendication 14 ou 15, dans lequel le au moins un élément de renforcement ou de rigidification (103) comprend une fibre optique ou un guide d'ondes (129).

**17.** Dispositif (100) pour utilisation invasive selon l'une quelconque des revendications 9 ou 10, dans lequel est prévu(e) au moins une fibre optique ou un guide d'ondes (129) sur l'intérieur de l'élément de support 101.

**18.** Dispositif (100) pour utilisation invasive selon l'une quelconque des revendications 9, 10 ou 17, dans lequel le au moins un composant électronique ou système microélectromécanique (200, 201) est placé adjacent à une extrémité frontale (107) de l'élément de support (101) et en contact opérationnel avec la au moins une ligne ou impression conductrice électriquement (117) ou ladite au moins une fibre optique ou ledit au moins un guide d'onde (129).

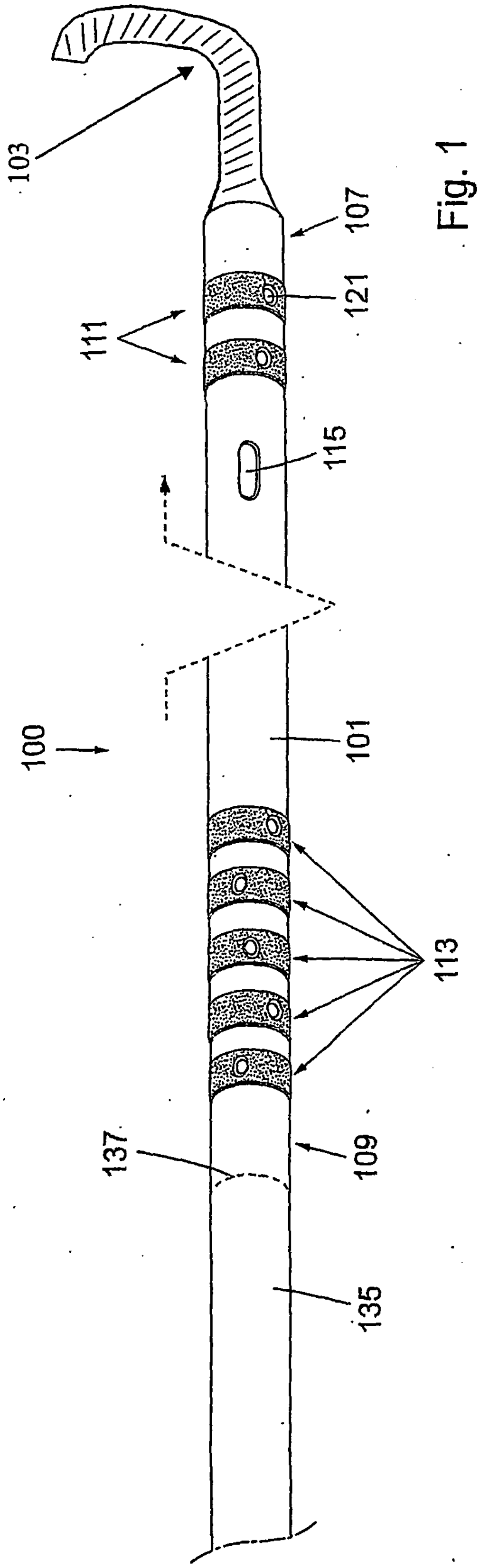


Fig. 1

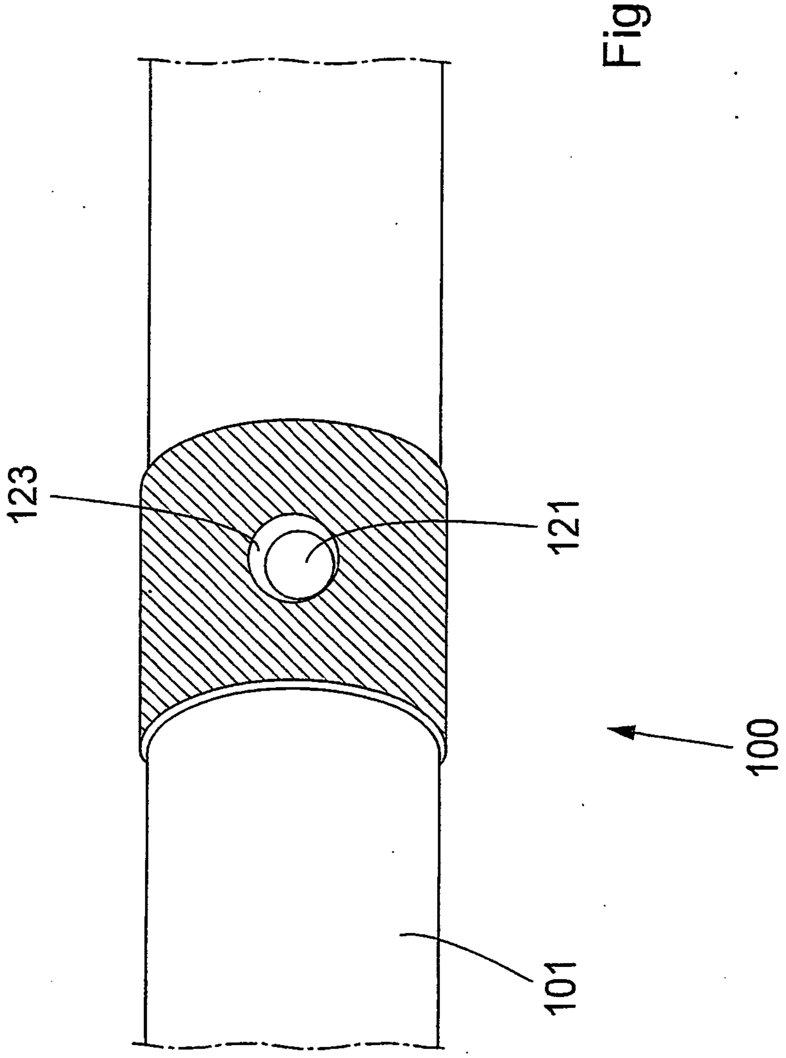


Fig. 2

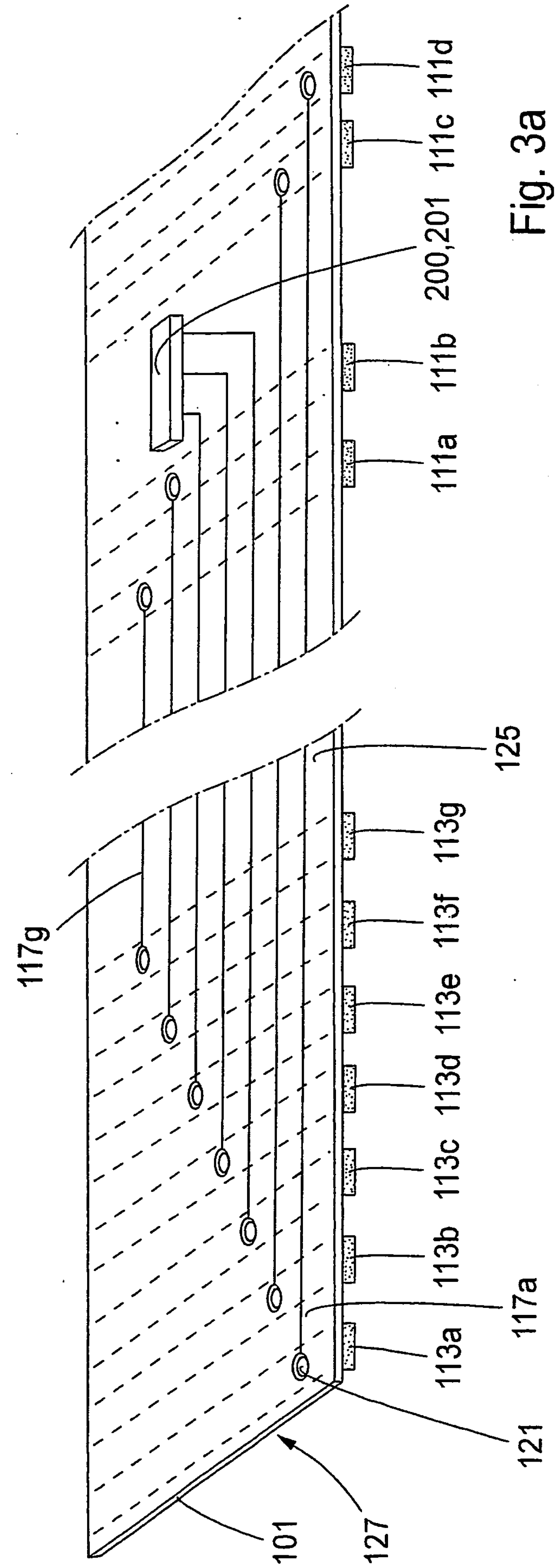
111d
111c
200,201
111b
111a
125
117g
113g
113f
113e
113d
113c
113b
113a
117a
121
101
127

Fig. 3a

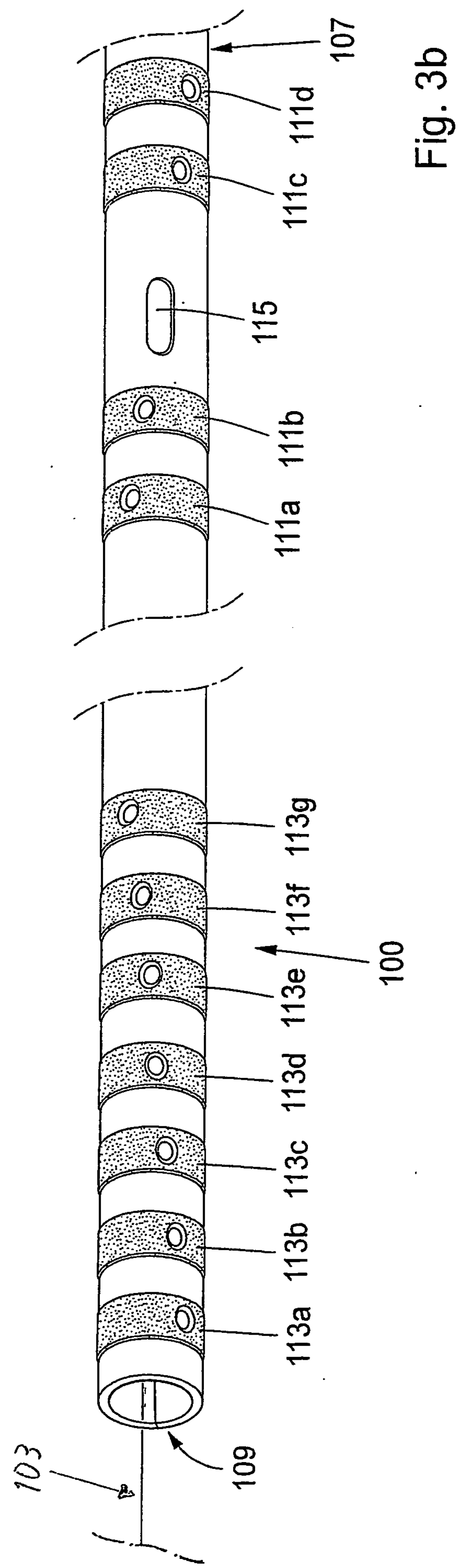
107
111d
111c
115
111b
111a
113g
113f
113e
113d
113c
113b
113a
100
109
103

Fig. 3b

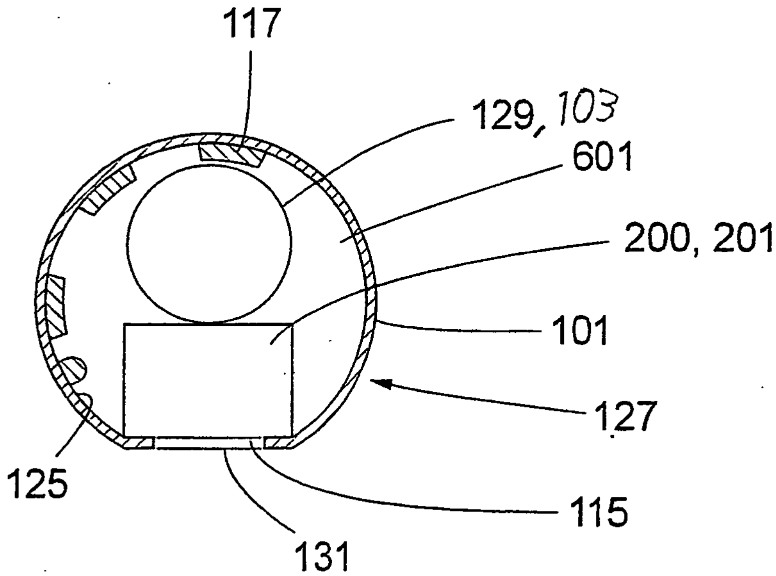


Fig. 4

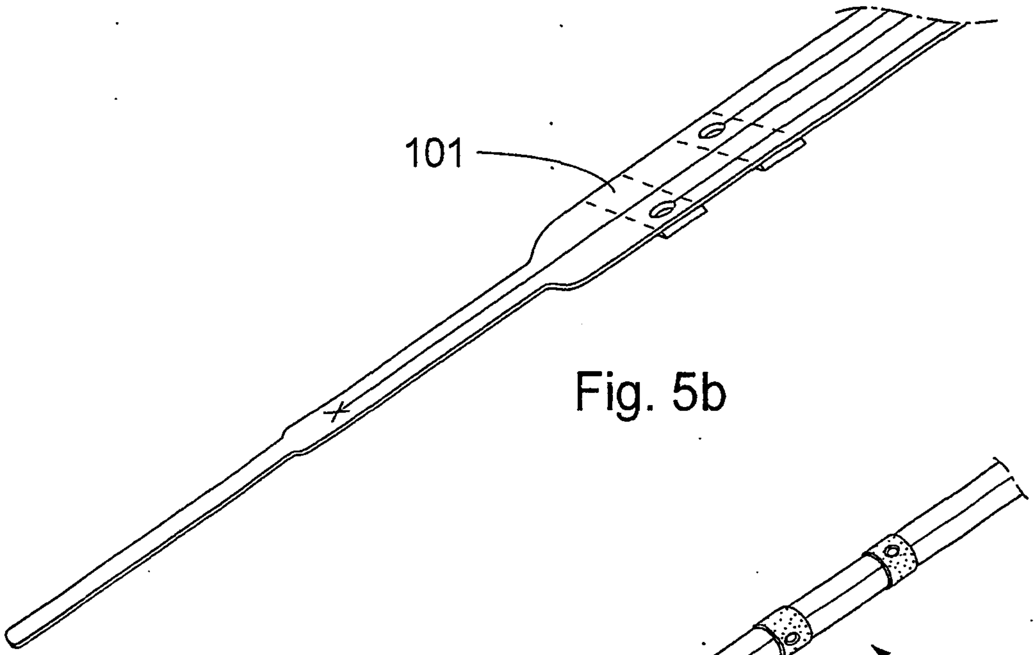


Fig. 5b

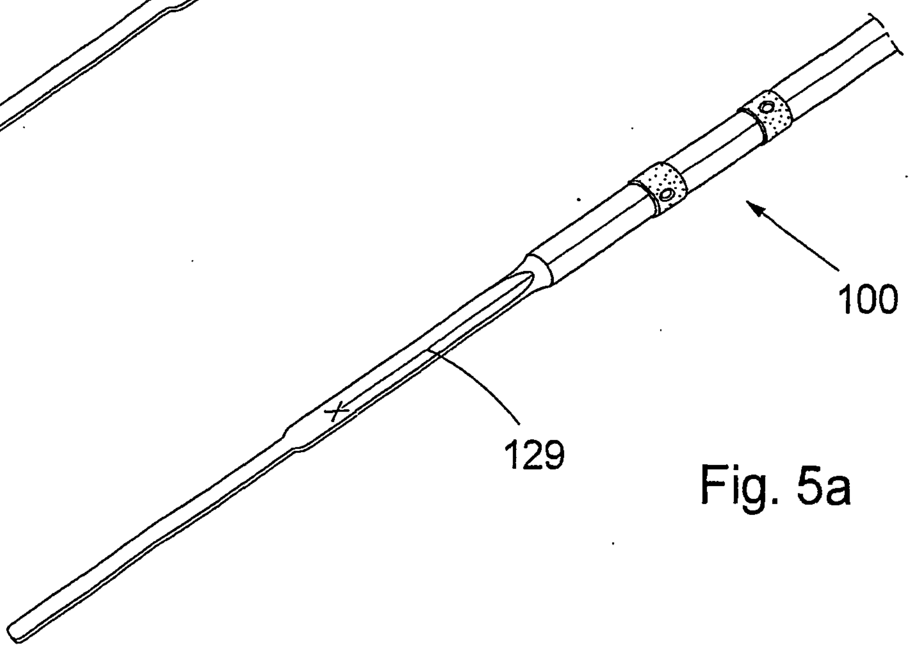


Fig. 5a

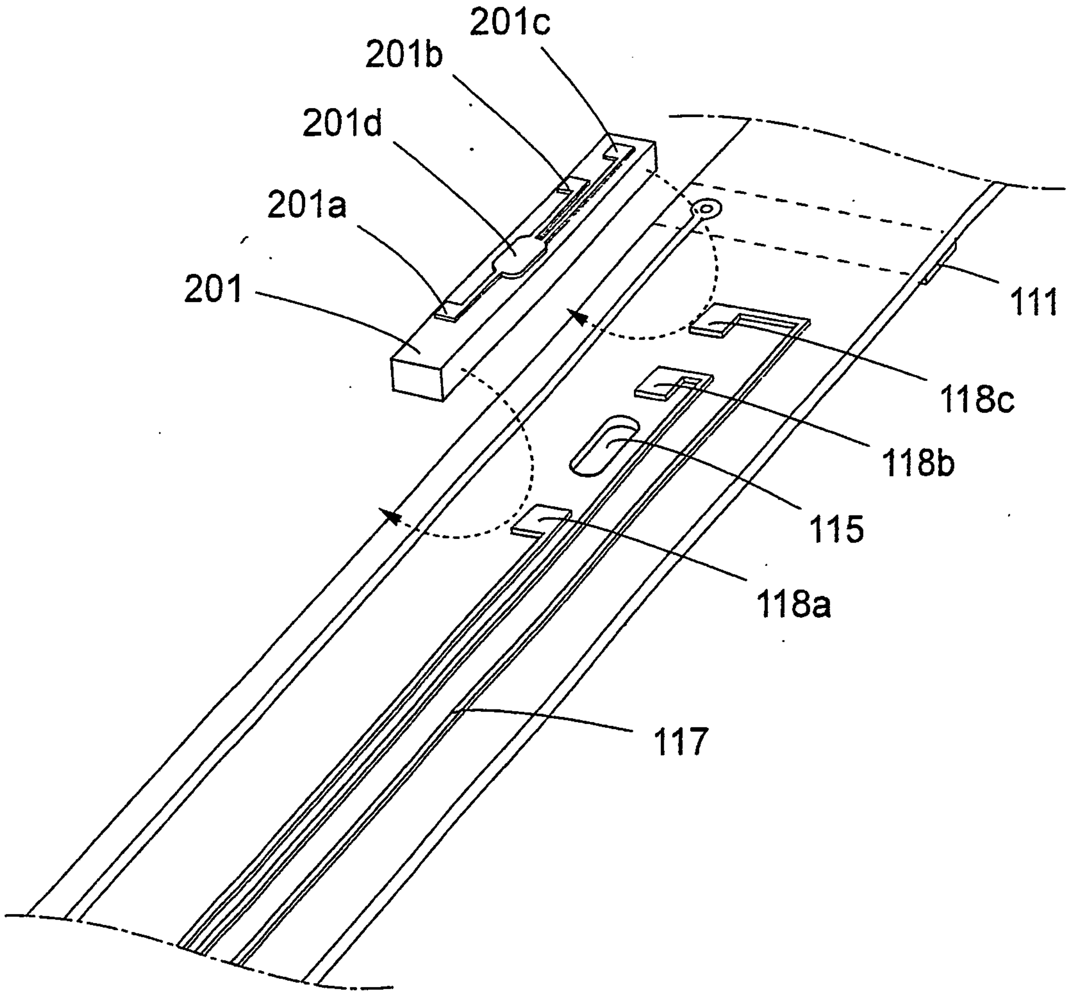


Fig. 6a

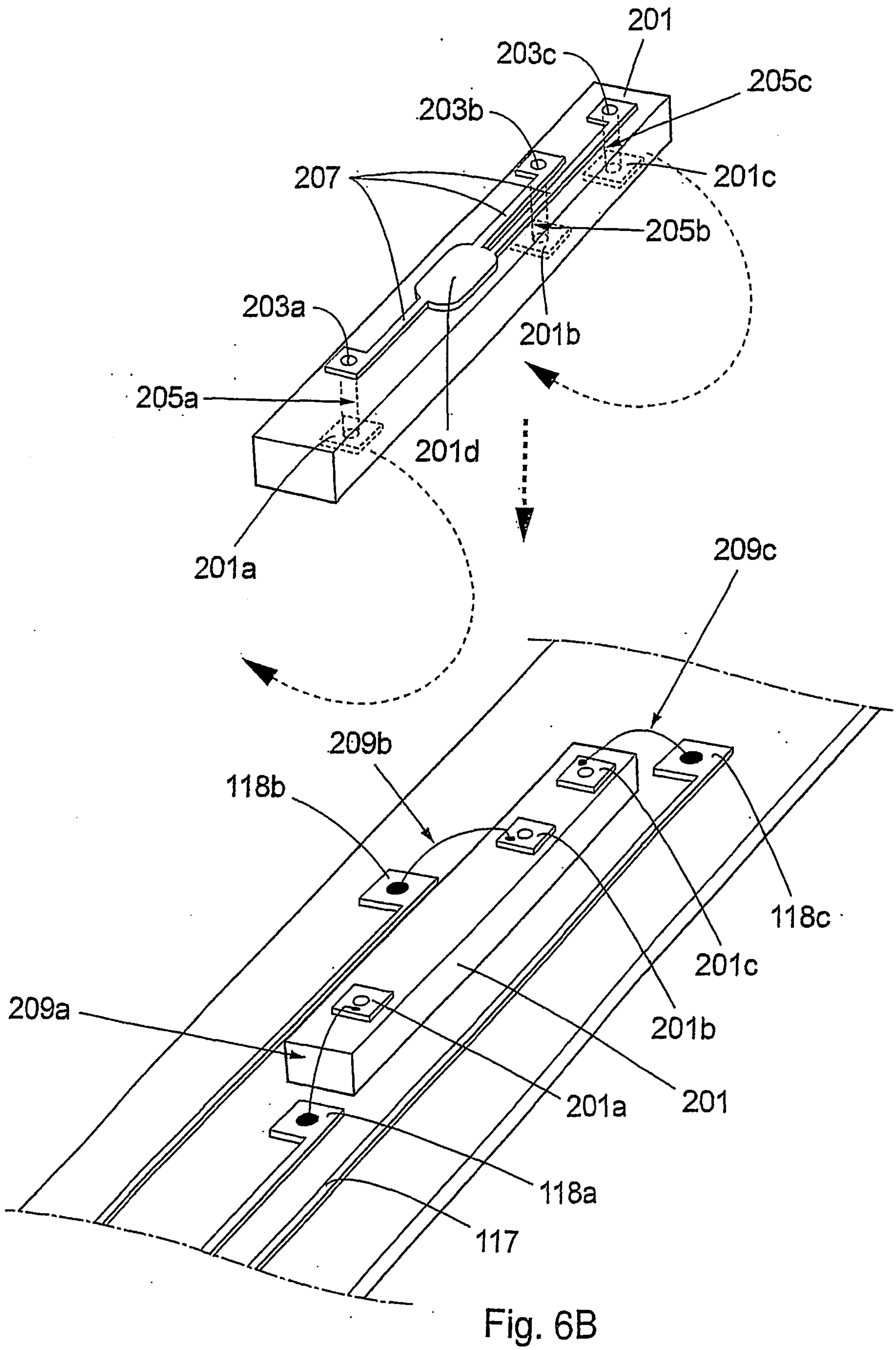
201
203c
205c
203b
207
201c
205b
203a
201b
205a
201d
201a
209c
209b
118b
118c
201c
209a
201b
201a
201
118a
117

Fig. 6B

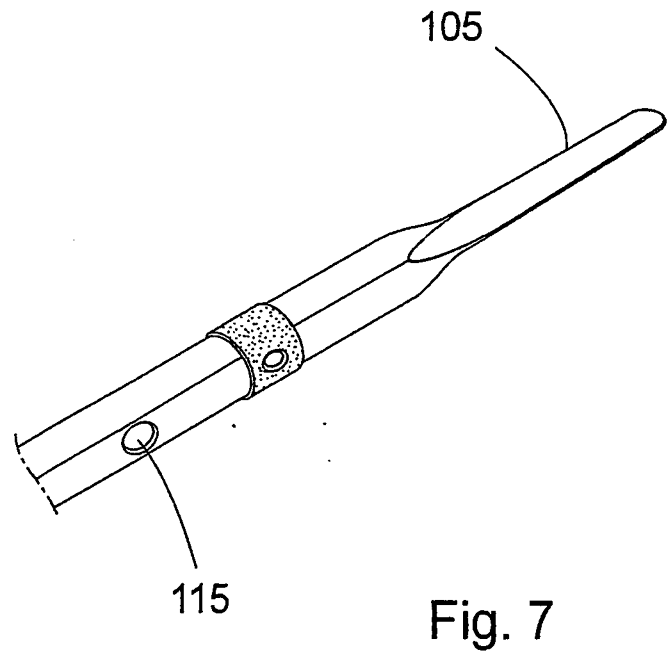


Fig. 7

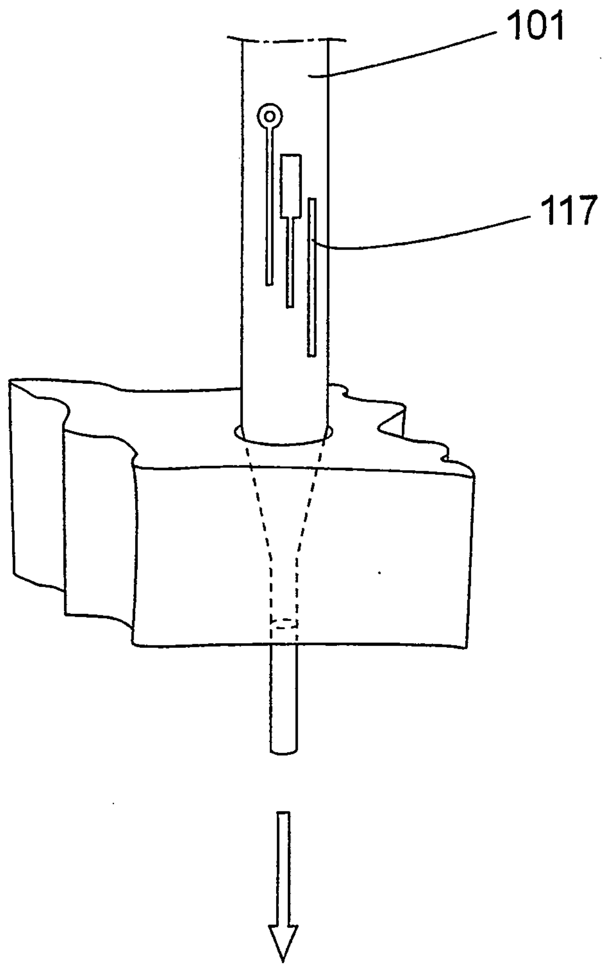


Fig. 8

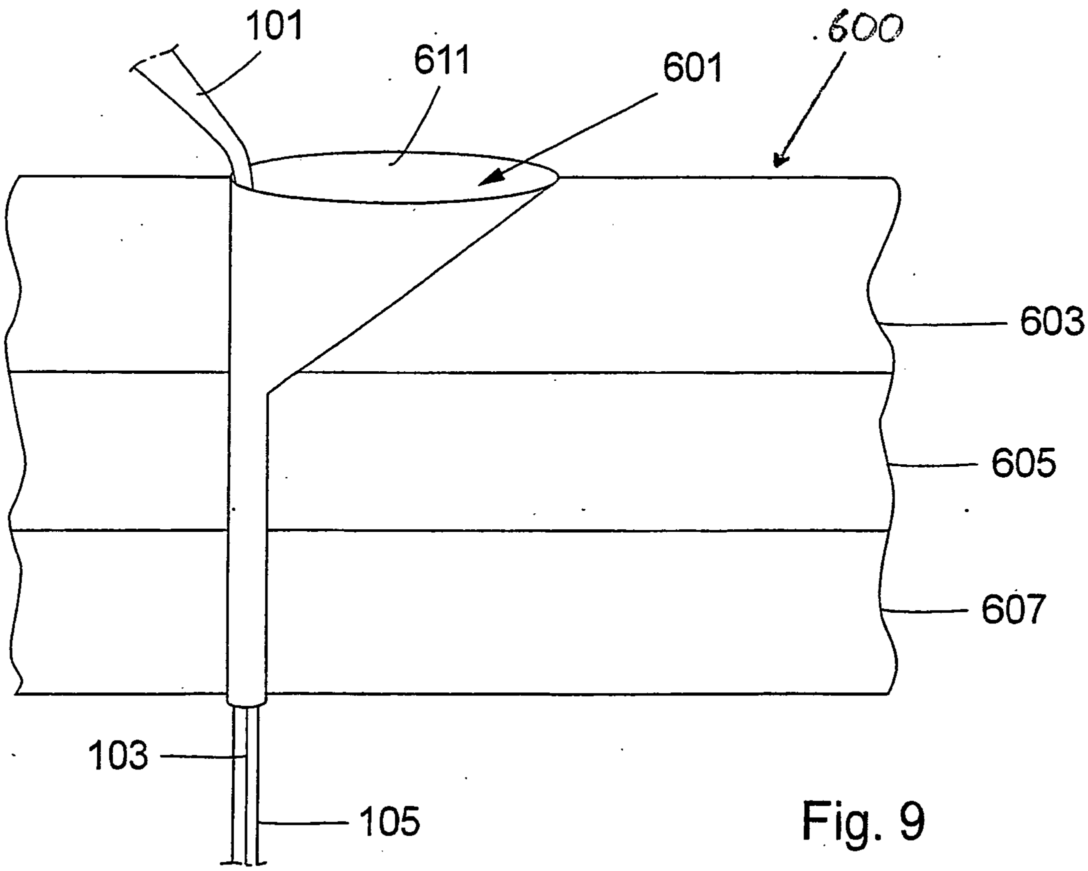


Fig. 9

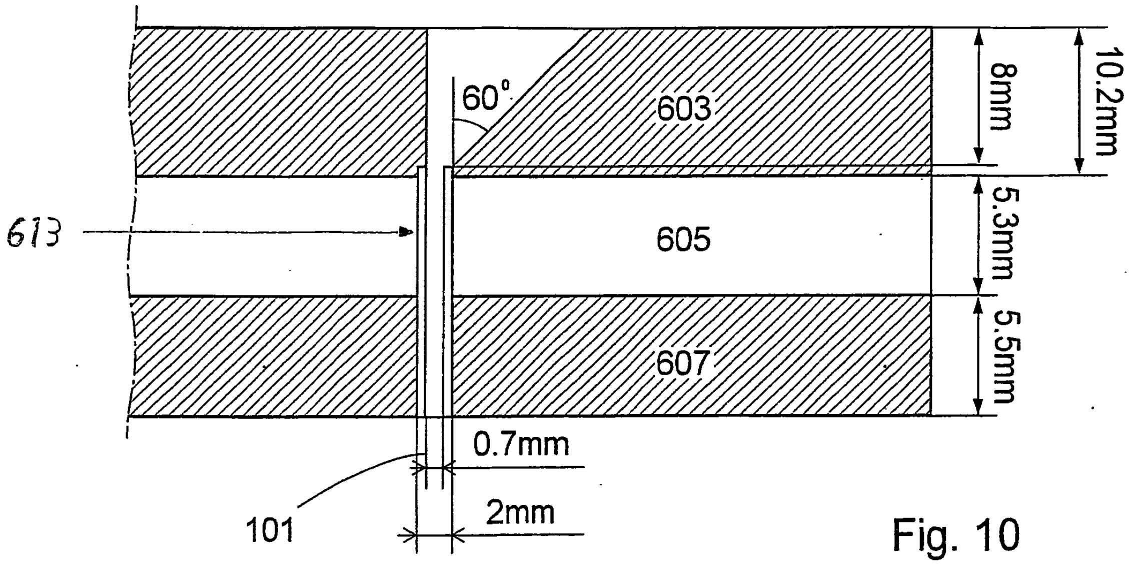


Fig. 10

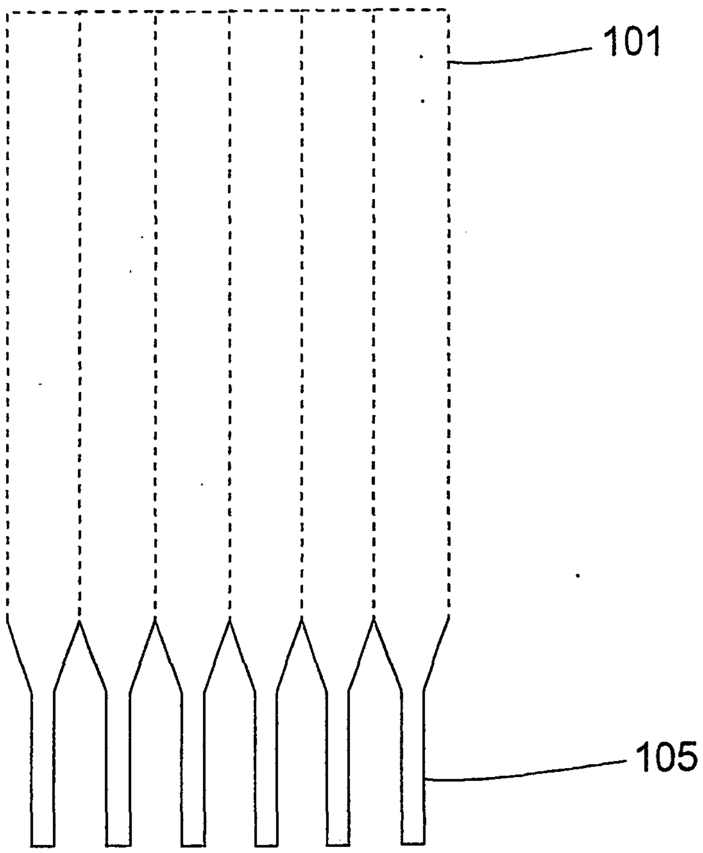


Fig. 11

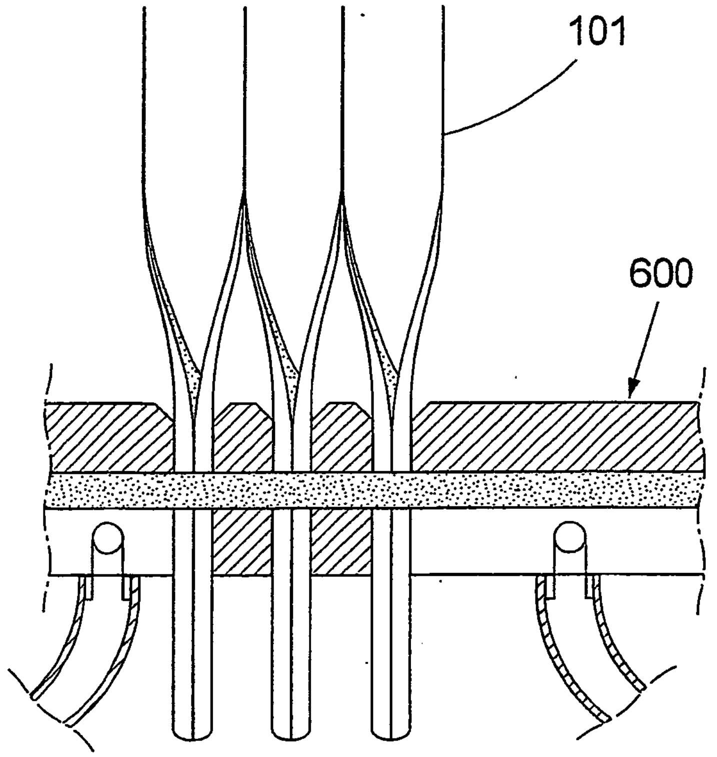


Fig. 12

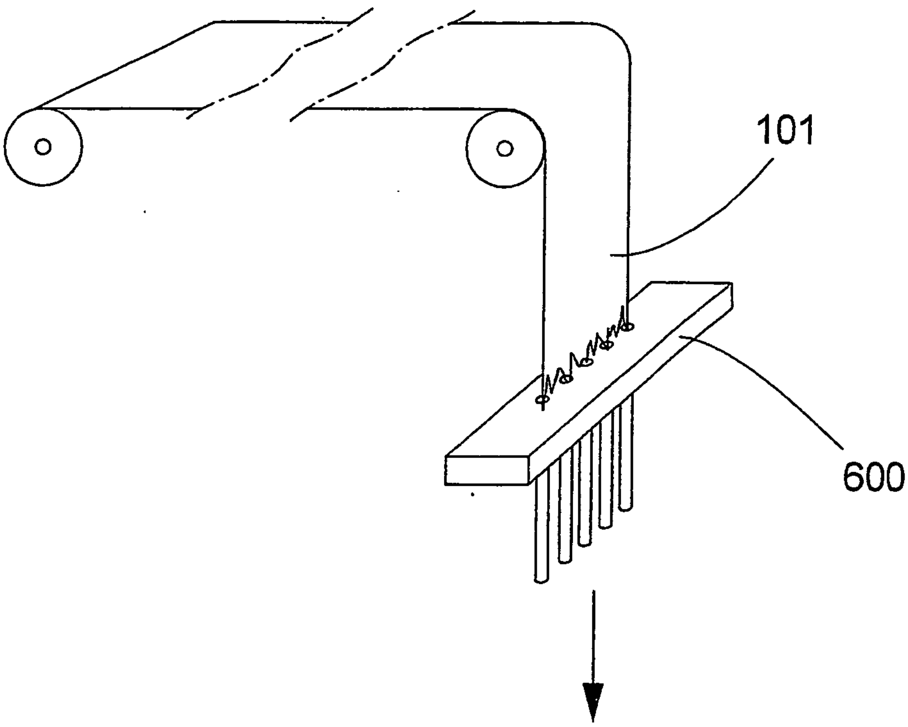


Fig. 13

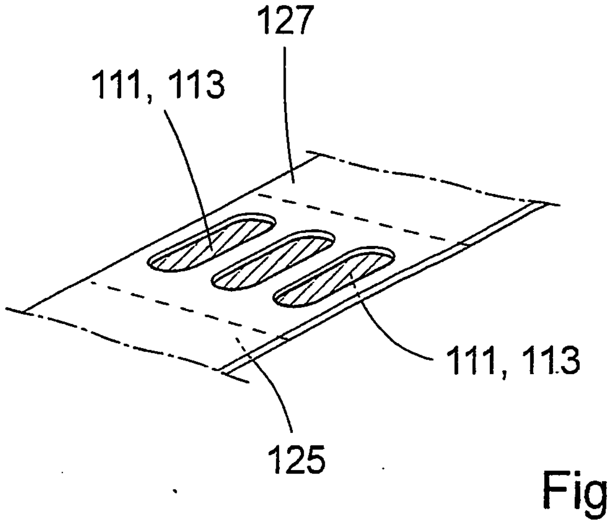


Fig. 14

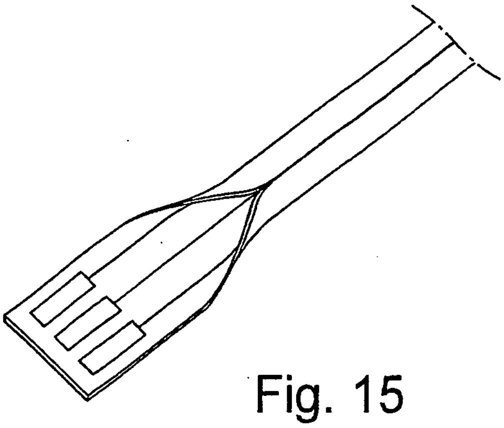

Fig. 15

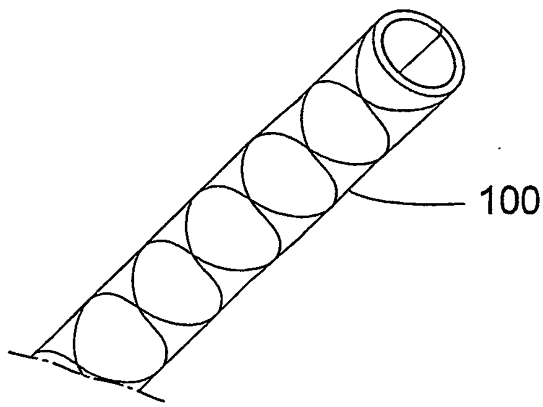


Fig. 16

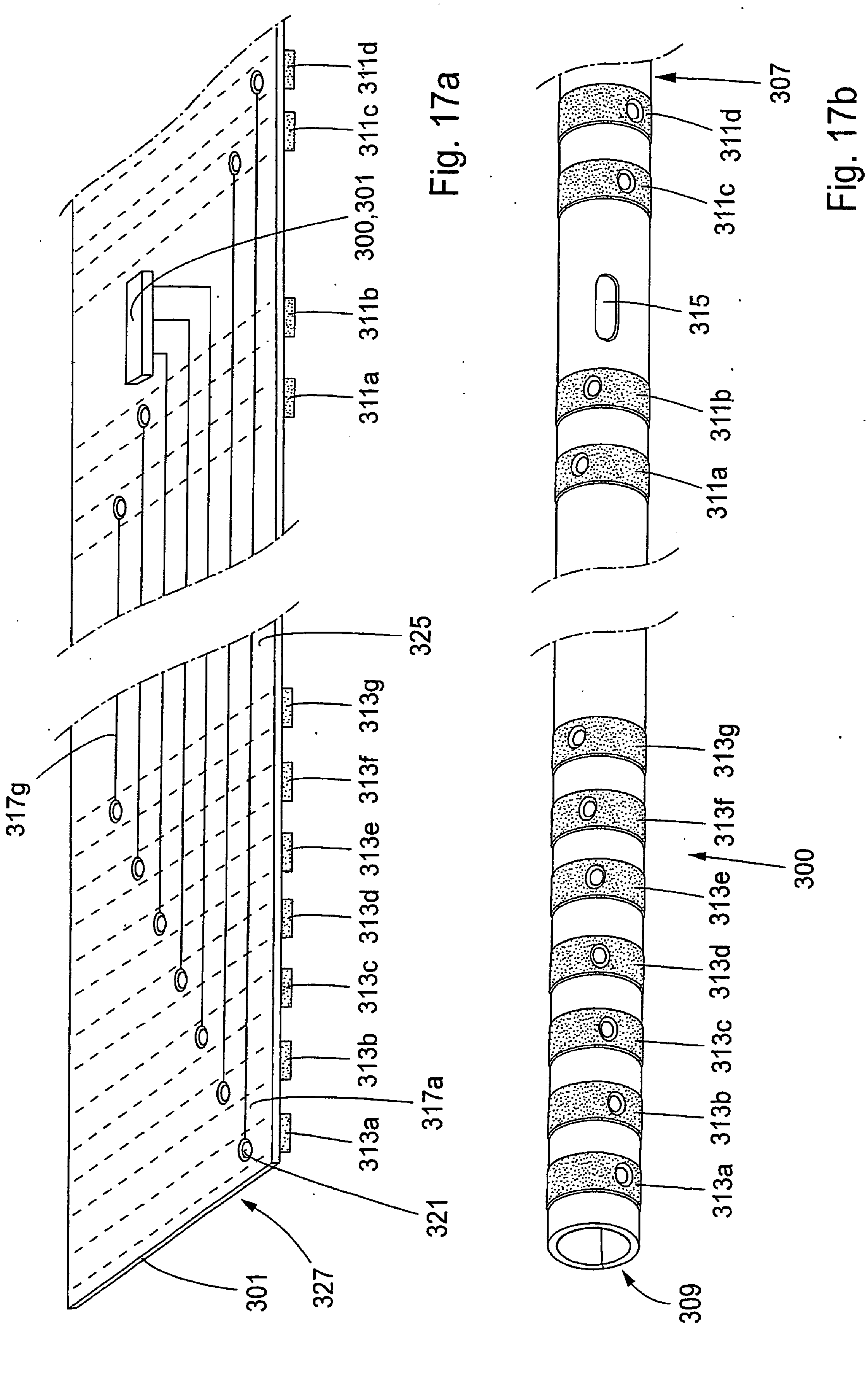


Fig. 17a

Fig. 17b

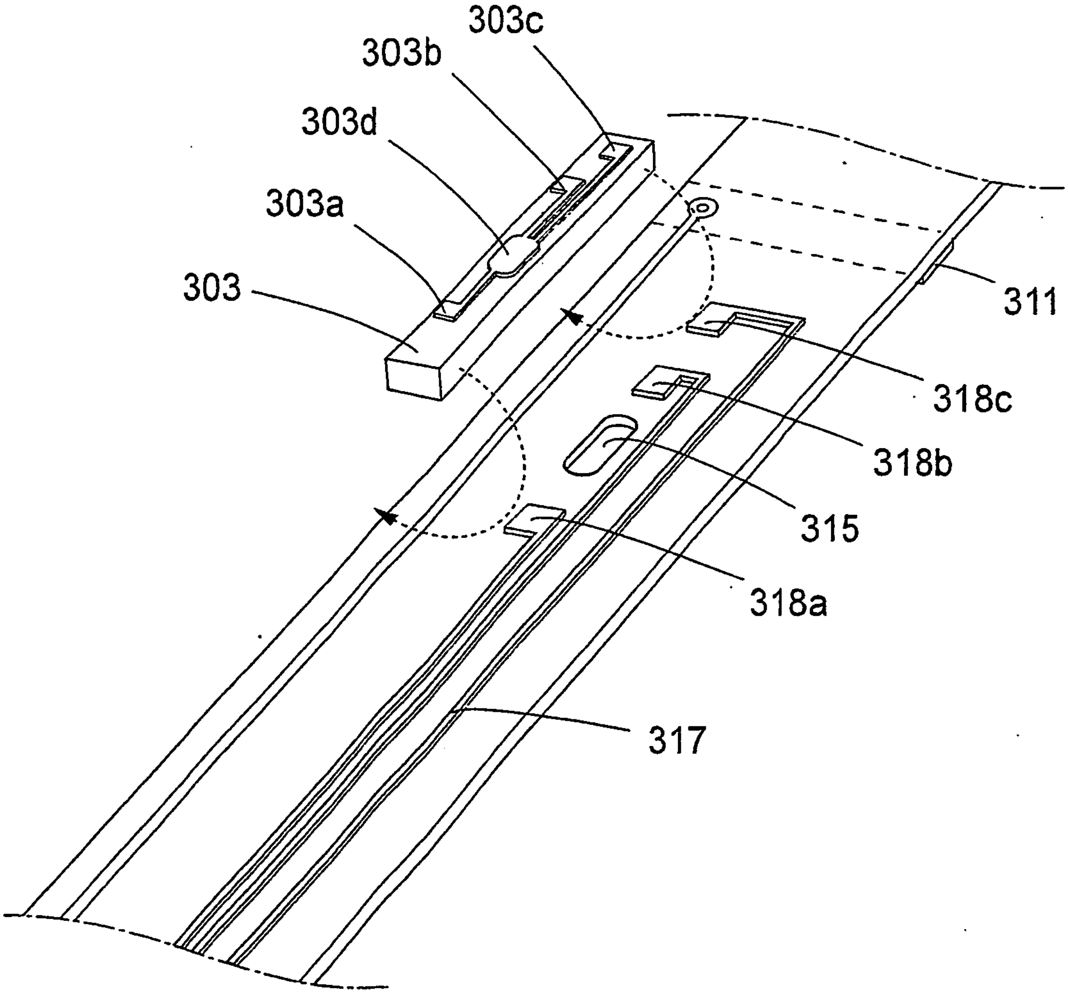


Fig. 18

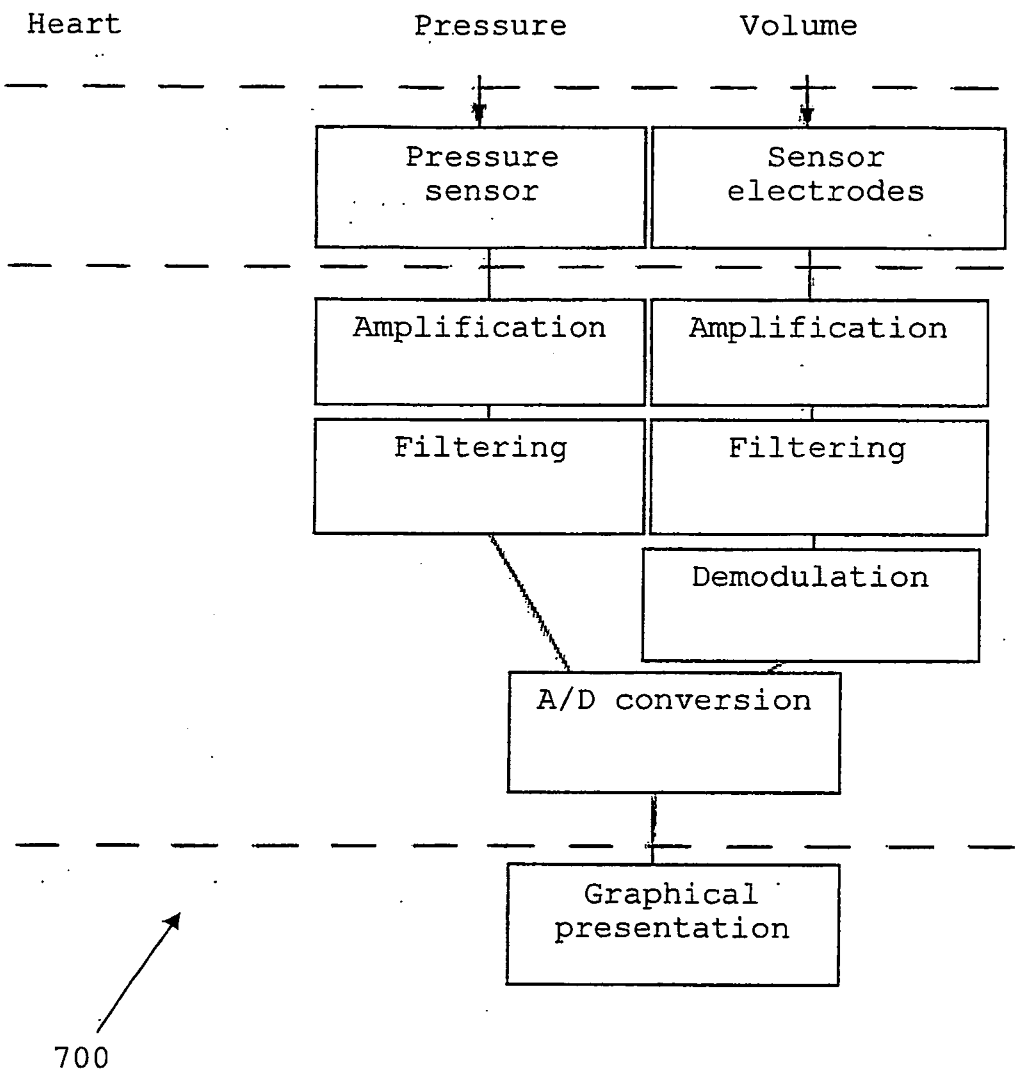
Heart
Pressure
Volume
Pressure sensor
Sensor electrodes
Amplification
Amplification
Filtering
Filtering
Demodulation
A/D conversion
Graphical presentation
700

Fig. 19

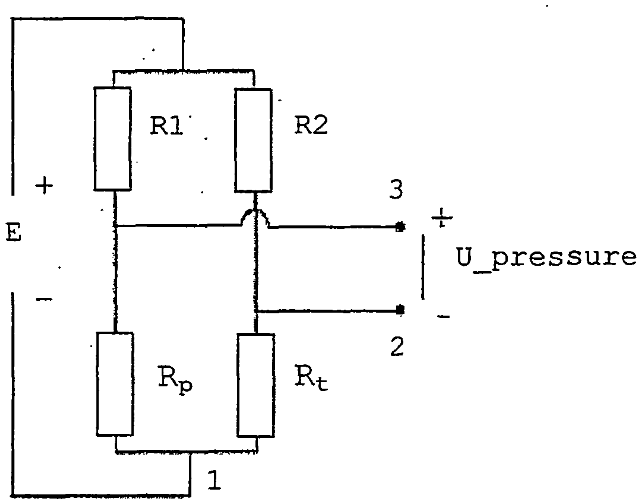


Fig. 20

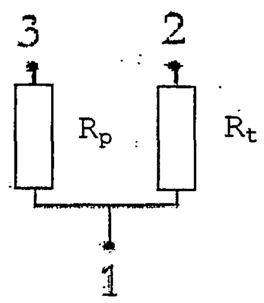


Fig. 21

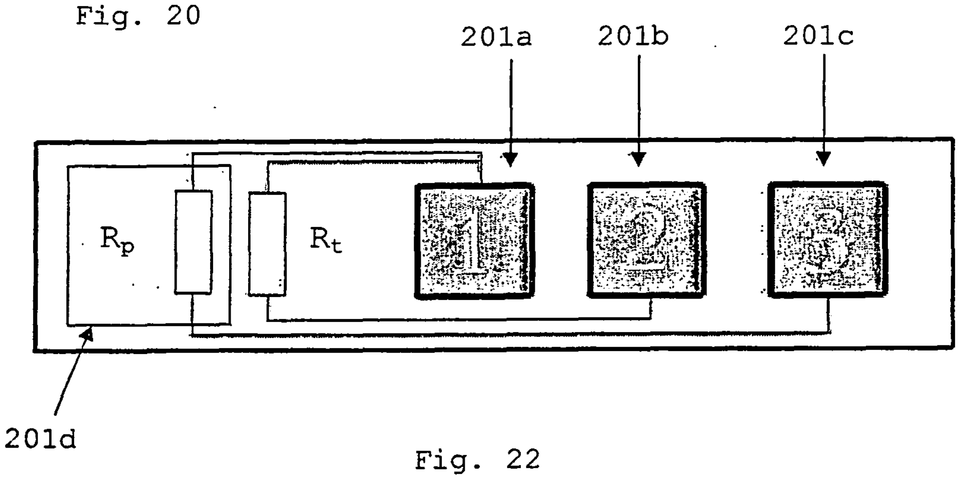


Fig. 22

Fig. 23a, Switching with the squarewave +0

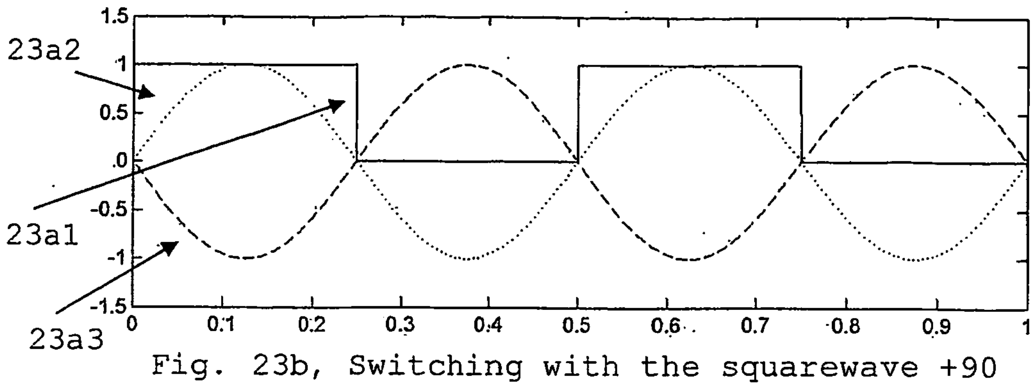


Fig. 23b, Switching with the squarewave +90

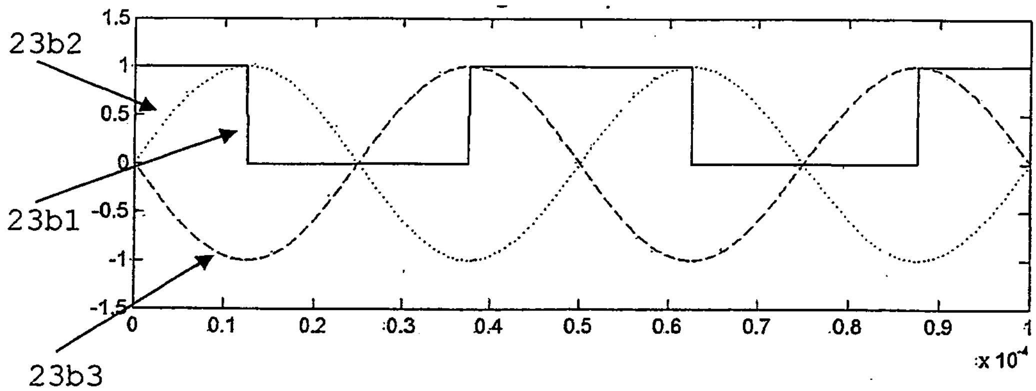

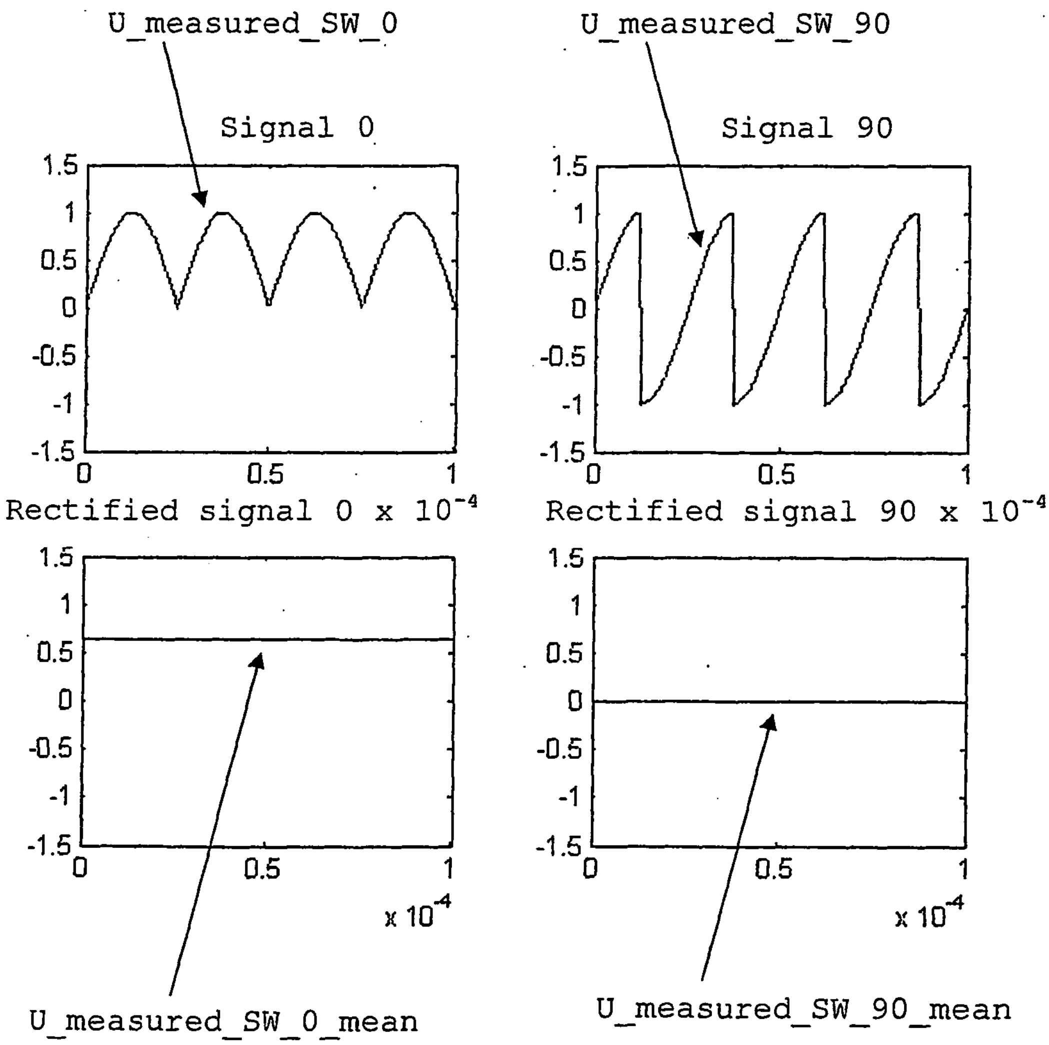


Fig. 24a (top left)
Fig. 24b (top right)
Fig. 24c (bottom left)
Fig. 24d (bottom right)

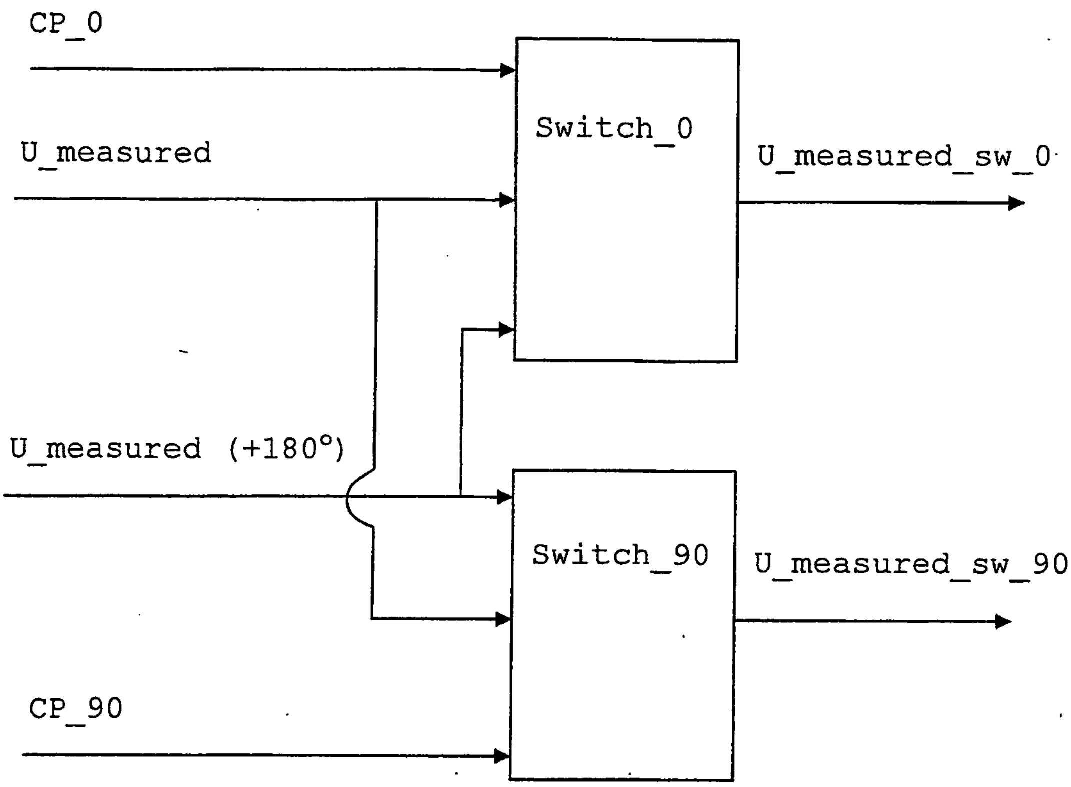
CP_0
U_measured
Switch_0
U_measured_sw_0
U_measured (+180°)
Switch_90
U_measured_sw_90
CP_90

Fig. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1714610 A1
- US 4762135 A
- US 5199433 A1
- US 5902330 A
- US 2005038320 A


### Non-patent literature cited in the description


- **INGELIN CLAUSEN ; OLA SVEEN.** Die separation and packaging of a surface micromachined piezoresistive pressure sensor. *Sensors and Actuators A: Physical,* 12 February 2007, vol. 133 (2), 457-466
- **BAAN J.** Continuous stroke volume and cardiac output from intra-ventricular dimensions obtained with impedance catheter. *Cardivascular Research,* 1981, vol. 15, 328-334
- **EMIL SÖDERQVIST.** New approaches to monitoring of cardiac function. Division of Medical Engineering, 2006